# EUROPEAN PATENT SPECIFICATION

(11) **EP 2 390 268 B1**
(45) Date of publication and mention of the grant of the patent: **01.11.2017**
(21) Application number: 10182757.4
(22) Date of filing: 07.11.2003
(51) Int. Cl.: C07K 16/28, C07K 16/18, C07K 16/46, A61K 39/395, C12N 15/13, G01N 33/577, A61P 37/06, A61P 19/02

(54) **Single domain antibodies directed against tumour necrosis factor-alpha and uses therefor**
Gegen Tumornekrosefaktor-alpha gerichtete Einzeldomänen-Antikörper und Verwendungen dafür
Anticorps à domaine unique dirigés contre le facteur de nécrose tumorale-alpha et leurs utilisations

(30) Priority: 08.11.2002 US 425073 P; 08.11.2002 US 425063 P; 10.01.2003 EP 03447005; 23.06.2003 WO PCT/EP03/06581; 08.07.2003 WO PCT/EP03/07313
(43) Date of publication of application: 30.11.2011
(62) Divisional of application: 03776676.3
(73) Proprietor: Ablynx N.V., 9052 Ghent-Zwijnaarde (BE)
(72) Inventor: Silence, Karen, 3090 Overijse (BE); Lauwereys, Marc, 9450 Haaltert (BE); De Haard, Hans, 4436 NA Oudelande (NL)
(74) Representative: Hoffmann Eitle

(56) References cited:
- WO-A1-91/02078
- ELS CONRATH K ET AL: "Camel single-domain antibodies as modular building units in bispecific and bivalent antibody constructs", JOURNAL OF BIOLOGICAL CHEMISTRY, AMERICAN SOCIETY OF BIOLOGICAL CHEMISTS, BALTIMORE, MD, US, vol. 276, no. 10, 9 March 2001 (2001-03-09), pages 7346-7350, XP002248402, ISSN: 0021-9258
- CORTEZ-RETAMOZO V ET AL: "Efficient tumor targeting by single-domain antibody fragments of camels", INTERNATIONAL JOURNAL OF CANCER, NEW YORK, NY, US, vol. 98, no. 3, 20 March 2002 (2002-03-20) , pages 456-462, XP002248403, ISSN: 0020-7136
- VALLE E ET AL: "Infliximab", EXPERT OPINION ON PHARMACOTHERAPY, ASHLEY, LONDON, UK, vol. 2, 1 June 2001 (2001-06-01), pages 1015-1025, XP002965315, ISSN: 1465-6566, DOI: DOI:10.1517/14656566.2.6.1015
- TANHA J ET AL: "Selection by phage display of llama conventional VH fragments with heavy chain antibody VHH properties", JOURNAL OF IMMUNOLOGICAL METHODS, ELSEVIER, AMSTERDAM, NL, vol. 263, no. 1-2, 1 May 2002 (2002-05-01) , pages 97-109, XP004354388, ISSN: 0022-1759

## Description

### FIELD OF THE INVENTION

The present invention provides polypeptides comprising at least two single domain antibodies directed towards tumor necrosis factor alpha (TNF-alpha) and at least one single domain antibody directed against a serum protein, wherein said serum protein is any of serum albumin, serum immunoglobulins, thyroxine-binding protein, transferrin, or fibrinogen. The present invention further relates to their use in therapy. Such antibodies may have a framework sequence with high homology to the human framework sequences. Compositions comprising antibodies to tumor necrosis factor alpha (TNF-alpha) alone or in combination with other drugs are described.

### BACKGROUND TO THE INVENTION

Tumor necrosis factor alpha (TNF-alpha) is believed to play an important role in various disorders, for example in inflammatory disorders such as rheumatoid arthritis, Crohn's disease, ulcerative colitis and multiple sclerosis. Both TNF-alpha and the receptors (CD120a, CD120b) have been studied in great detail. TNF-alpha in its bioactive form is a trimer and the groove formed by neighboring subunits is important for the cytokine-receptor interaction. Several strategies to antagonize the action of the cytokine have been developed and are currently used to treat various disease states.
A TNF-alpha inhibitor which has sufficient specificity and selectivity to TNF-alpha may be an efficient prophylactic or therapeutic pharmaceutical compound for preventing or treating disorders where TNF-alpha has been implicated as causative agent. WO 91/02078 describes conventional four chain antibodies raised against TNF-alpha. Methods of treating toxic shock (EP 486526), tumor regression, inhibition of cytotoxicity (US 6448380, US 6451983, US 6498237), autoimmune disease such as RA and Crohn's disease (EP 663836, US 5672347, US 5656272), graft versus host reaction (US 5672347), bacterial meningitis (EP 585705) by means of an antibody to TNF-alpha have been described.

Yet none of the presently available drugs are completely effective for the treatment of autoimmune disease, and most are limited by severe toxicity. In addition, it is extremely difficult and a lengthy process to develop a new chemical entitiy (NCE) with sufficient potency and selectivity to such target sequence. Antibody-based therapeutics on the other hand have significant potential as drugs because they have exquisite specificity to their target and a low inherent toxicity. In addition, the development time can be reduced considerably when compared to the development of new chemical entities (NCE's). However, conventional antibodies are difficult to raise against multimeric proteins where the receptor-binding domain of the ligand is embedded in a groove, as is the case with TNF-alpha. Heavy chain antibodies described in the invention which are derived from *Camelidae,* are known to have cavity-binding propensity (WO97/49805; Lauwereys et al, EMBO J. 17, 5312, 1998)). Therefore, such heavy chain antibodies are inherently suited to bind to receptor binding domains of such ligands as TNF. In addition, such antibodies are known to be stable over long periods of time, therefore increasing their shelf-life (Perez et al, Biochemistry, 40, 74, 2001). Furthermore, such heavy chain antibody fragments can be produced 'en-masse' in fermentors using cheap expression systems compared to mammalian cell culture fermentation, such as yeast or other microorganisms (EP 0 698 097).

The use of antibodies derived from sources such as mouse, sheep, goat, rabbit etc., and humanised derivatives thereof as a treatment for conditions which require a modulation of inflammation is problematic for several reasons. Traditional antibodies are not stable at room temperature, and have to be refrigerated for preparation and storage, requiring necessary refrigerated laboratory equipment, storage and transport, which contribute towards time and expense. Refrigeration is sometimes not feasible in developing countries. Furthermore, the manufacture or small-scale production of said antibodies is expensive because the mammalian cellular systems necessary for the expression of intact and active antibodies require high levels of support in terms of time and equipment, and yields are very low. Furthermore the large size of conventional antibodies, would restrict tissue penetration, for example, at the site of inflamed tissue. Furthermore, traditional antibodies have a binding activity which depends upon pH, and hence are unsuitable for use in environments outside the usual physiological pH range such as, for example, in treating gastric bleeding, gastric surgery. Furthermore, traditional antibodies are unstable at low or high pH and hence are not suitable for oral administration. However, it has been demonstrated that camelidae antibodies resist harsh conditions, such as extreme pH, denaturing reagents and high temperatures (Dumoulin et al, Protein Science 11, 500, 2002), so making them suitable for delivery by oral administration. Furthermore, traditional antibodies have a binding activity, which depends upon temperature, and hence are unsuitable for use in assays or kits performed at temperatures outside biologically active-temperature ranges (*e.g*. 37 ± 20°C).

Polypeptide therapeutics and in particular antibody-based therapeutics have significant potential as drugs because they have exquisite specificity to their target and a low inherent toxicity. However, it is known by the skilled addressee that an antibody which has been obtained for a therapeutically useful target requires additional modification in order to prepare it for human therapy, so as to avoid an unwanted immunological reaction in a human individual upon administration thereto. The modification process is commonly termed "humanisation". It is known by the skilled artisan that antibodies raised in species, other than in humans, require humanisation to render the antibody therapeutically useful in humans ( (1) CDR grafting : Protein Design Labs: US 6180370, US 5693761; Genentech US 6054297; Celltech: 460167, EP 626390, US 5859205; (2) Veneering: Xoma: US 5869619, US 5766886, US 5821123). There is a need for a method for producing antibodies which avoids the requirement for substantial humanisation, or which completely obviates the need for humanisation. There is a need for a new class of antibodies which have defined framework regions or amino acid residues and which can be administered to a human subject without the requirement for substantial humanisation, or the need for humanisation at all.

Another important drawback of conventional antibodies is that they are complex, large molecules and therefore relatively unstable, and they are sensitive to breakdown by proteases. This means that conventional antibody drugs cannot be administered orally, sublingually, topically, nasally, vaginally, rectally or by inhalation because they are not resistant to the low pH at these sites, the action of proteases at these sites and in the blood and/or because of their large size. They have to be administered by injection (intravenously, subcutaneously, etc.) to overcome some of these problems. Administration by injection requires specialist training in order to use a hypodermic syringe or needle correctly and safely. It further requires sterile equipment, a liquid formulation of the therapeutic polypeptide, vial packing of said polypeptide in a sterile and stable form and, of the subject, a suitable site for entry of the needle. Furthermore, subjects commonly experience physical and psychological stress prior to and upon receiving an injection. Therefore, there is need for a method for the delivery of therapeutic polypeptides which avoids the need for injection which is not only cost/time saving, but which would also be more convenient and more comfortable for the subject.

Single domain antibody-based therapeutics have significant potential as drugs because they have exquisite specificity to their target and a low inherent toxicity. However, improving further their intrinsic and functional affinity can lead to many benefits for a patient such as reduced dose of therapeutic, faster therapy, and reduced side effects.

### THE AIMS OF THE PRESENT INVENTION

It is an aim of the present invention is to provide polypeptides comprising one or more single domain antibodies which bind to TNF-alpha, homologues of said polypeptides, functional portions of homologues of said polypeptides. Said polypeptides modify the biological activity of TNF-alpha upon binding. Such polypeptides might bind into the receptor-binding groove of TNF-alpha, or might not bind in the receptor binding groove. Such polypeptides are single domain antibodies.

It is a further aim of the present invention to provide single domain antibodies which may be any of the art, or any future single domain antibodies. Examples include, but are not limited to, heavy chain antibodies, antibodies naturally devoid of light chains, single domain antibodies derived from conventional 4-chain antibodies, engineered antibodies and single domain scaffoids other than those derived from antibodies. According to one aspect of the invention, a single domain antibody as used herein is a naturally occurring single domain antibody known as heavy chain antibody devoid of light chains (WO 9404678). For clarity reasons, this variable domain derived from a heavy chain antibody devoid of light chain will be called VHH or nanobody to distinguish it from the conventional VH of four chain immunoglobulins. Such a VHH molecule can be derived from antibodies raised in *Camelidae* species, for example in camel, Ilama, dromedary, alpaca and guanaco.

It is a further aim of the invention to provide a method of administering anti-TNF-alpha polypeptides intravenously, subcutaneously, orally, sublingually, topically, nasally, vaginally, rectally or by inhalation.

It is a further aim of the invention to enhance the binding affinity of monovalent single domain antibodies. The present invention is described in the appended claims.

### SUMMARY OF THE INVENTION

The present invention is described in the appended claims.

One embodiment of the present invention is an anti-TNF-alpha polypeptide comprising at least two anti-TNF-alpha single domain antibodies and at least one single domain antibody directed against a serum protein, wherein said serum protein is any of serum albumin, serum immunoglobulins, thyroxine-binding protein, transferrin, or fibrinogen.

Another embodiment of the present invention is an anti-TNF-alpha polypeptide as described above wherein at least one anti-TNF-alpha single domain antibody corresponds to a sequence represented by any of SEQ ID NOs: 1 to 16 and 79 to 84.

Another embodiment described herein is an anti-TNF-alpha polypeptide as described above further comprising at least one single domain antibody directed against a serum protein.

Another embodiment of the present invention is an anti-TNF-alpha polypeptide as described above wherein said serum protein is any of serum albumin, serum immunoglobulins, thyroxine-binding protein, transferring, or fibrinogen.

Another embodiment described herein is an anti-TNF-alpha polypeptide as described above wherein a single domain anti-serum protein single domain antibody correspond to a sequence represented by any of SEQ ID NOs: 26 to 29 and 85 to 97.

Another embodiment described herein is an anti-TNF-alpha polypeptide as described above corresponding to a sequence represented by any of SEQ ID NOs: 30 to 43.

Another embodiment of the present invention is an anti-TNF-alpha polypeptide as described above further comprising at least one single domain antibody selected from the group consisting of anti-IFN-gamma single domain antibody, anti-TNF-alpha receptor single domain antibody and anti-IFN-gamma receptor single domain antibody.

Another embodiment described herein is an anti-TNF-alpha polypeptide as described above, wherein the number of single domain antibodies directed against TNF-alpha is at least two.

Another embodiment described herein is an anti-TNF-aipha polypeptide as described above corresponding to a sequence represented by any of SEQ ID NOs: 73 to 76.

Another embodiment of the present invention is an anti-TNF-alpha polypeptide as described above, wherein at least one single domain antibody is a humanized Camelidae VHHs.

Another embodiment described herein is an anti-TNF-alpha polypeptide as described above wherein a humanized Camelidae VHH corresponds to a sequence represented by any of SEQ ID NOs: 17 to 19 and 21 to 24.

Another embodiment described herein is a composition comprising an anti-TNF-alpha polypeptide as described above and at least one single domain antibody from the group consisting of anti-IFN-gamma single domain antibody, anti-TNF-alpha receptor single domain antibody and anti-IFN-gamma receptor single domain antibody, for simultaneous, separate or sequential administration to a subject.

Another embodiment described herein is an anti-TNF-alpha polypeptide as described above, or a composition as described above wherein at least one anti-IFN-gamma single domain antibody correspond to a sequence represented by any of SEQ ID NOs: 44 to 72.

Another embodiment of the present invention is an anti-TNF-alpha polypeptide as described above, or a composition as described above, wherein said single domain antibody is an homologous sequence, a functional portion, or a functional portion of an homologous sequence of the full length single domain antibody.

Another embodiment of the present invention is an anti-TNF-alpha polypeptide as described above, or a composition as described above, wherein the anti-TNF-alpha polypeptide is an homologous sequence, a functional portion, or a functional portion of an homologous sequence of the full length anti-TNF-alpha polypeptide.

Another embodiment of the present invention is an anti-TNF-alpha polypeptide as described above, or a composition as described above wherein at least one single domain antibody is a Camelidae VHH.

Another embodiment of the present invention is a nucleic acid encoding an anti-TNF-alpha polypeptide as described above.

Another embodiment described herein is a method of identifying an agent that modulates the binding of an anti-TNF-alpha polypeptide as described above, to Tumor Necrosis Factor-alpha comprising the steps of:
(a) contacting an anti-TNF-alpha polypeptide as described above with a target that is Tumor Necrosis Factor alpha, in the presence and absence of a candidate modulator under conditions permitting binding between said polypeptide and target, and
(b) measuring the binding between the polypeptide and target of step (a), wherein a decrease in binding in the presence of said candidate modulator, relative to the binding in the absence of said candidate modulator identified said candidate modulator as an agent that modulates the binding of an anti-TNF-alpha polypeptide as described above and Tumor Necrosis Factor-alpha.

Another embodiment described herein is a method of identifying an agent that modulates Tumor Necrosis Factor-alpha-mediated disorders through the binding of an anti-TNF-alpha polypeptide as described above to Tumor Necrosis Factor-alpha comprising:
(a) contacting an anti-TNF-alpha polypeptide as described above with a target that is Tumor Necrosis Factor alpha, in the presence and absence of a candidate modulator under conditions permitting binding between said polypeptide and target, and
(b) measuring the binding between the polypeptide and target of step (a), wherein a decrease in binding in the presence of said candidate modulator, relative to the binding in the absence of said candidate modulator identified, said candidate modulator as an agent that modulates Tumor Necrosis Factor alpha-mediated disorders.

Another embodiment described herein is a method of identifying an agent that modulates the binding of Tumor Necrosis Factor alpha to its receptor through the binding of an anti-TNF-alpha polypeptide as described above to Tumor Necrosis Factor-alpha comprising:
(a) contacting an anti-TNF-alpha polypeptide as described above with a target that is Tumor Necrosis Factor-alpha, in the presence and absence of a candidate modulator under conditions permitting binding between said polypeptide and target, and
(b) measuring the binding between the polypeptide and target of step (a), wherein a decrease in binding in the presence of said candidate modulator, relative to the binding in the absence of said candidate modulator identified said candidate modulator as an agent that modulates the binding of Tumor Necrosis Factor-alpha to its receptor.

Another embodiment described herein is a kit for screening for agents that modulate Tumor Necrosis Factor-alpha-mediated disorders comprising an anti-TNF-alpha polypeptide as described above and Tumor Necrosis Factor-alpha.

Another embodiment described herein is an unknown agent that modulates the binding of an anti-TNF-alpha polypeptide as described above to Tumor Necrosis Factor-alpha, identified according to the method as described above.

Another embodiment described herein is an unknown agent that modulates Tumor Necrosis Factor-alpha-mediated disorders, identified according to the methods as described above.

Another embodiment described herein is an unknown agent as described above wherein said disorders are one or more of inflammation, rheumatoid arthritis, Crohn's disease, ulcerative colitis, inflammatory bowel syndrome and multiple sclerosis.

Another embodiment of the present invention is an anti-TNF-alpha polypeptide as described above, or a nucleic acid as described above for use in a method of treating and/or preventing and/or alleviating disorders relating to inflammatory processes.

Another embodiment of the present invention is a use of an anti-TNF-alpha polypeptide as described above or a nucleic acid as described above, or a composition as described above for the preparation of a medicament for treating and/or preventing and/or alleviating disorders relating to inflammatory reactions.

Another embodiment of the present invention is an anti-TNF-alpha polypeptide as described above or a composition as described above, for use in a method of treating and/or preventing and/or alleviating disorders susceptible to modulation by a TNF-alpha modulating substance which is able pass through the gastric environment without the substance being inactivated.

Another embodiment of the present invention is an use of an anti-TNF-aipha polypeptide as described above or a composition as described above, for the preparation of a medicament for treating, preventing and/or alleviating the symptoms of disorders susceptible to modulation by a TNF-alpha modulating substance which is able pass through the gastric environment without the substance being inactivated.

Another embodiment of the present invention is an anti-TNF-alpha polypeptide as described above or a composition as described above, for use in a method of treating and/or preventing and/or alleviating disorders susceptible to modulation by a TNF-alpha modulating substance delivered to the vaginal and/or rectal tract.

Another embodiment of the present invention is a use of an anti-TNF-alpha polypeptide as described above or a composition as described above, for the preparation of a medicament for treating, preventing and/or alleviating the symptoms of disorders susceptible to modulation by a TNF-alpha modulating substance delivered to the vaginal and/or rectal tract.

Another embodiment of the present invention is an anti-TNF-alpha polypeptide as described above or a composition as described above, for use in a method of treating and/or preventing and/or alleviating disorders susceptible to modulation by a TNF-alpha modulating substance delivered to the nose, upper respiratory tract and/or lung.

Another embodiment of the present invention is a use of an anti-TNF-alpha polypeptide as described above or a composition as described above, for the preparation of a medicament for treating, preventing and/or alleviating the symptoms of disorders susceptible to modulation by a TNF-alpha modulating substance delivered to the nose, upper respiratory tract and/or lung.

Another embodiment of the present invention is an anti-TNF-alpha polypeptide as described above or a composition as described above, for use in a method of treating and/or preventing and/or alleviating disorders susceptible to modulation by a TNF-alpha modulating substance delivered to the intestinal mucosa, wherein said disorder increases the permeability of the intestinal mucosa.

Another embodiment of the present invention is a use of an anti-TNF-alpha polypeptide as described above or a composition as described above, for the preparation of a medicament for treating, preventing and/or alleviating the symptoms of disorders susceptible to modulation by a TNF-alpha modulating substance delivered to the intestinal mucosa, wherein said disorder increases the permeability of the intestinal mucosa.

Another embodiment of the present invention is an anti-TNF-alpha polypeptide as described above or a composition as described above, for use in a method of treating and/or preventing and/or alleviating disorders susceptible to modulation by a TNF-alpha modulating substance which is able pass through the tissues beneath the tongue effectively.

Another embodiment of the present invention is a use of an anti-TNF-alpha polypeptide as described above or a composition as described above, for the preparation of a medicament for treating, preventing and/or alleviating the symptoms of disorders susceptible to modulation by a TNF-alpha modulating substance which is able pass through the tissues beneath the tongue effectively.

Another embodiment described herein is an anti-TNF-alpha polypeptide as described above or a composition as described above, for treating and/or preventing and/or alleviating disorders susceptible to modulation by a TNF-alpha modulating substance which is able pass through the skin effectively.

Another embodiment described herein is a use of an anti-TNF-alpha polypeptide as described above or a composition as described above, for the preparation of a medicament for treating, preventing and/or alleviating the symptoms of disorders susceptible to modulation by a TNF-alpha modulating substance which is able pass through the skin effectively.

Another embodiment of the present invention is a nucleic acid as described above or, an anti-TNF-alpha polypeptide as described above for use in a method for treating and/or preventing and/or alleviating disorders relating to inflammatory processes wherein said disorders are any of inflammation, rheumatoid arthritis, Crohn's disease, ulcerative colitis, inflammatory bowel syndrome, multiple sclerosis, Addison's disease, Autoimmune hepatitis, Autoimmune parotitis, Diabetes Type I, Epididymitis, Glomerulonephritis, Graves' disease, Guillain-Barre syndrome, Hashimoto's disease, Hemolytic anemia, Systemic lupus erythematosus, Male infertility, Multiple sclerosis, Myasthenia Gravis, Pemphigus, Psoriasis, Rheumatic fever, Rheumatoid arthritis, Sarcoidosis, Scleroderma, Sjogren's syndrome, Spondyloarthropathies, Thyroiditis, and Vasculitis.

Another embodiment of the present invention is a composition comprising a nucleic acid as described above, an anti-TNF-alpha polypeptide as described above and a suitable pharmaceutical vehicle.

Another embodiment described herein is a method of diagnosing a disorder characterised by the dysfunction of Tumor Necrosis Factor-alpha comprising:
(a) contacting a sample with an anti-TNF-alpha polypeptide as described above,
(b) detecting binding of said polypeptide to said sample, and
(c) comparing the binding detected in step (b) with a standard, wherein a difference in binding relative to said sample is diagnostic of a disorder characterised by dysfunction of Tumor Necrosis Factor-alpha.

Another embodiment described herein is a kit for screening for a disorder as cited above, using a method as described above.

Another embodiment described herein is a kit for screening for a disorder as cited above comprising an isolated anti-TNF-alpha polypeptide as described above.

Another embodiment described herein is a use of an anti-TNF-aipha polypeptide as described above for the purification of said Tumor Necrosis Factor-alpha.

Another embodiment of the present invention is a use of an anti-TNF-alpha polypeptide as described above for inhibiting the interaction between Tumor Necrosis Factor-alpha and one or more Tumor Necrosis Factor-alpha receptors.

Another embodiment described herein is a method for producing an anti-TNF-alpha polypeptide as described above comprising the steps of:
(a) obtaining double stranded DNA encoding a Camelidae VHH directed to Tumor Necrosis Factor alpha,
(b) cloning and expressing the DNA selected in step (b).

Another embodiment described herein is a method of producing an anti-TNF-alpha polypeptide as described above comprising:
(a) culturing host cells comprising nucleic acid capable of encoding an anti-TNF-alpha polypeptide as described above, under conditions allowing the expression of the polypeptides, and,
(b) recovering the produced polypeptide from the culture.

Another embodiment described herein is a method as described above, wherein said host cells are bacterial or yeast.

Another embodiment described herein is a kit for screening for any of inflammation, rheumatoid arthritis, Crohn's disease, ulcerative colitis, inflammatory bowel syndrome or multiple sclerosis comprising an anti-TNF-alpha polypeptide as described above.

### BRIEF DESCRIPTION OF FIGURES AND TABLES

**Figure 1** Alignment of anti-human TNF VHH's as described in Example 1
**Figure 2** Dilution series of anti-human TNF-alpha VHHs as tested in ELISA according to Example 1.
**Figure 3** Antagonistic effect of VHH as determined in cytotoxicity assay using human cell line KYM according to Example 1.
**Figure 4** In vitro receptor binding assay of wild type VHH#12B and mutant A74S+Y76N+K83R+P84A
**Figure 5** In vitro receptor binding assay of wild type VHH#12B and mutant 1E + Q5LA74S + Y76N + K83R + P84A
**Figure 6** Binding in ELISA of wild type VHH#3E and mutant VHH's
**Figure 7** In vitro receptor binding assay of wild type VHH#3E and mutant VHH's
**Figure 8** Alignment of antagonistic anti-mouse TNF's as described in Example 3.
**Figure 9** Antagonistic effect of anti-mouse TNF VHH as determined in cytotoxicity assay using murine cell line L929 according to Example 3.
**Figure 10** EcoRI - HindIII insert of vector pAX11 (pUC119 backbone) for production of bivalent or bispecific VHH.
**Figure 11** Coomassie-stained PAGE (15%) of IMAC-purified mono-(lane 8), bi- (lane 1), tri- (lanes 2, 3 and 5) and tetravalent (lanes 4, 6 and 7) anti- TNFa VHH.
**Figure 12** Chromatogram of the analysis by gel filtration on Superdex 75HR of the mono-, bi-, tri and tetravalent VHH.
**Figure 13** Comparison of the antagonistic characteristics of the mono-, bi-, tri- and tetravalent form of the anti-human TNF VHH with the clinically used products Remicade and Enbrel.
**Figure 14** Antagonistic behaviour of the mono- and bivalent VHH's directed against mouse TNFalpha.
**Figure 15** Coomassie stained PAGE of VHH-Fc-fusion derived from human IgG1 described in Example 4.
**Figure 16** Antagonistic efficacy of VHH-Fc fusion derived from VHH#3E compared with bivalent format of VHH#3E as determined in bioassay.
**Figure 17** ELISA of reference and pepsin-treated TNF3E at pH2.2, pH3.2 and pH4.2 (100% is the signal measured at a 1/100 dilution)
**Figure 18** Experimental setting
**Table 1** Amino acid sequence listing of the peptides of aspects of present invention directed against TNF-alpha.
**Table 2** List of mutagenesis reactions, mutagenic primers and templates used for mutagenesis of VHH#12B.
**Table 3** List of mutagenesis reactions, mutagenic primers and templates used for mutagenesis of VHH#3E.
**Table 4** Overview of humanised and wild type VHH
**Table 5** Anti-mouse serum albumin/anti TNF-alpha
**Table 6** Amino acid sequence listing of VHH's directed against human IFN-gamma.
**Table 7** Sequences of bivalent (BIV 3E, BIV#m3F), trivalent (TRI3E) or tetravalent (TETRA 3E) VHH directed against TNF-alpha.
**Table 8** Fractional homologies between the amino acid sequences of anti-mouse serum albumin VHHs of the invention.
**Table 9** Fractional homologies between anti-TNF-alpha VHHs of the invention.
**Table 10** Percentage homologies between anti-IFN-gamma VHHs of the invention.
**Table 11** Treatment schedule

### DETAILED DESCRIPTION

The present invention relates to an anti-tumour necrosis factor-alpha (TNF-alpha) polypeptide, comprising at least two single domain antibodies which are directed against TNF-alpha and at least one single domain antibody directed against a serum protein, wherein said serum protein is any of serum albumin, serum immunoglobulins, thyroxine-binding protein, transferrin, or fibrinogen. The invention also relates to nucleic acids capable of encoding said polypeptides.

Single domain antibodies are antibodies whose complementary determining regions are part of a single domain polypeptide. Examples include, but are not limited to, heavy chain antibodies, antibodies naturally devoid of tight chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, bovine. According to one aspect of the invention, a single domain antibodies as used herein is a naturally occurring single domain antibody known as heavy chain antibody devoid of light chains. Such single domain antibodies are disclosed in WO 94/04678 for example. For clarity reasons, this variable domain derived from a heavy chain antibody naturally devoid of light chain is known herein as a *VHH* or *nanobody* to distinguish it from the conventional VH of four chain immunoglobulins. Such a VHH molecule can be derived from antibodies raised in *Camelidae* species, for example in camel, dromedary, llama, alpaca and guanaco. Other species besides *Camelidae* may produce heavy chain antibodies naturally devoid of light chain; such VHHs are within the scope of the invention.

VHHs, according to the present invention, and as known to the skilled addressee are heavy chain variable domains derived from immunoglobulins naturally devoid of light chains such as those derived from *Camelidae* as described in WO 94/04678 (and referred to hereinafter as VHH domains or nanobodies). VHH molecules are about 10x smaller than IgG molecules. They are single polypeptides and very stable, resisting extreme pH and temperature conditions. Moreover, they are resistant to the action of proteases which is not the case for conventional antibodies. Furthermore, *in vitro* expression of VHHs produces high yield, properly folded functional VHHs. In addition, antibodies generated in *Camelids* will recognize epitopes other than those recognised by antibodies generated in *vitro* through the use of antibody libraries or via immunisation of mammals other than *Camelids* (WO 9749805). As such, anti-TNF-alpha VHH's may interact more efficiently with TNF-alpha than conventional antibodies, thereby blocking its interaction with the TNF-alpha receptor more efficiently.

According to the invention, TNF-alpha is derived from any species. Examples of species relevant to the invention include as rabbits, goats, mice, rats, cows, calves, camels, llamas, monkeys, donkeys, guinea pigs, chickens, sheep, dogs, cats, horses, and preferably humans.

TNF-alpha is also a fragment of TNF-alpha, capable of eliciting an immune response. TNF-alpha is also a fragment of TNF-alpha, capable of binding to a single domain antibody raised against the full length TNF-alpha.

A single domain antibody directed against TNF-alpha means single domain antibody that it is capable of binding to TNF-alpha with an affinity of better than 10⁻⁶ M.

One embodiment of the present invention is an anti-TNF polypeptide, wherein the single domain antibodies comprise *Camelidae* VHH directed against TNF-alpha.

The at least two single domain antibodies of the anti-TNF polypeptide which are directed against a TNF-alpha may be of the same sequence. Alternatively they may not all have the same sequence. It is within the scope of the invention that an anti-TNF polypeptide comprises anti-TNF-alpha single domain antibodies which do not all share the same sequence, but which are directed against the same target, one or more antigens thereof.

Another embodiment of the present invention is an anti-TNF-alpha polypeptides, wherein a single domain antibody corresponds to a sequence represented by any of SEQ ID NOs: 1 to 16 and 79 to 84 as shown in Table 1. Said sequences are derived from *Camelidae* heavy chain antibodies (VHHs) which are directed against TNF-alpha.

The present invention further relates to an anti-TNF-alpha polypeptide, wherein said single domain antibody is a VHH directed against TNF-alpha, wherein the VHH belongs to a class having human-like sequences. The class is characterised in that the VHHs carry an amino acid from the group consisting of glycine, alanine, valine, leucine, isoleucine, proline, phenylalanine, tyrosine, tryptophan, methionine, serine, threonine, asparagine, or glutamine at position 45, such as, for example, L45 and a tryptophan at position 103, according to the Kabat numbering. The new class of *Camelidae* single-domain antibodies described in this invention (Table 1, Example 1) is represented by VHH#2B (SEQ ID NO: 3) and VHH#12B (SEQ ID No. 14) containing the hydrophobic residues in FR2 in combination with the hydrophobic residue tryptophan at position 103.

Another human-like class of *Camelidae* single domain antibodies represented by sequences VHH#1A (SEQ ID NO. 1), VHH#4B (SEQ ID NO. 12), VHH#8-29 (SEQ ID NO. 81), VHH#8-41 (SEQ ID NO. 82), VHH#8-42 (SEQ ID NO. 83) and VHH#8-44 (SEQ ID NO. 84) (Table 1, Example 1) have been described in WO03035694 and contain the hydrophobic FR2 residues typically found in conventional antibodies of human origin or from other species, but compensating this loss in hydrophilicity by the charged arginine residue on position 103 that substitutes the conserved tryptophan residue present in VH from double-chain antibodies. As such, peptides belonging to these two classes show a high amino acid sequence homology to human VH framework regions and said peptides might be administered to a human directly without expectation of an unwanted immune response therefrom, and without the burden of further humanisation. The invention also relates to nucleic acids capable of encoding said polypeptides.

Therefore, one aspect of the present invention allows for the use in a method of administration of an anti-TNF-alpha polypeptide, wherein the single domain antibodies belong to the humanized class of VHH, and comprise a sequence represented by any of SEQ ID NO:1, 3, 12, 14, 81, 82, 83, and 84 to a patient in need of the same.

Any of the VHHs as used by the invention may be of the traditional class or of the classes of human-like *Camelidae* antibodies. Said antibodies may be directed against whole TNF-alpha or a fragment thereof, or a fragment of a homologous sequence thereof. These polypeptides include the full length *Camelidae* antibodies, namely Fc and VHH domains, chimeric versions of heavy chain *Camelidae* antibodies with a human Fc domain or VHH's by themselves or derived fragments.

Anti-serum albumin VHH's may interact in a more efficient way with serum albumin than conventional antibodies which is known to be a carrier protein. As a carrier protein some of the epitopes of serum albumin may be inaccessible by bound proteins, peptides and small chemical compounds. Since VHH's are known to bind into 'unusual' or nonconventional epitopes such as cavities (WO 97/49805), the affinity of such VHH's to circulating albumin may be increased.

The present invention also relates to the finding that an anti-TNF polypeptide as described herein further comprising one or more single domain antibodies directed against one or more serum proteins of a subject, surprisingly has significantly prolonged half-life in the circulation of said subject compared with the half-life of the anti-TNF-alpha single domain antibody when not part of said construct. Examples of such polypeptides are represented in Table 5 by SEQ ID NOs: 30 to 43. Furthermore, the said polypeptides were found to exhibit the same favourable properties of single domain antibodies such as high stability remaining intact in mice, extreme pH resistance, high temperature stability and high target affinity.

Another embodiment of the present invention is an anti-TNF-alpha polypeptide further comprising one or more single domain antibodies directed against one or more serum proteins, said anti-TNF alpha polypeptide comprising a sequence corresponding to any represented by SEQ ID NOs: 30 to 43 (Table 5).

Another embodiment of the present invention is an anti-TNF-alpha polypeptide, wherein an anti-serum protein single domain antibody corresponds to a sequence represented by any of SEQ ID NOs: 26 to 29 and 85 to 97 as shown in Table 5.

The serum protein may be any suitable protein found in the serum of subject. In one aspect of the invention, the serum protein is serum albumin, serum immunoglobulins, thyroxine-binding protein, transferrin, or fibrinogen. Depending on the intended use such as the required half-life for effective treatment and/or compartimentalisation of the target antigen, the VHH-partner can be directed to one of the above serum proteins.

Another aspect described herein is an anti-TNF-alpha polypeptide as disclosed herein further comprising at least one polypeptide selected from the group consisting of an anti-IFN-gamma polypeptide, an anti-TNF-alpha receptor polypeptide and anti-IFN-gamma receptor polypeptide.

It is an embodiment described herein that a single domain antibody directed against IFN-gamma corresponds to a sequence represented by any of SEQ ID NOs: 44 to 72 as shown in Table 6.

According to one aspect of the disclosure, a single domain antibody is directed against TNF-alpha receptor. Said single domain antibody may be a *Camelidae* VHH.

According to one aspect described herein, a single domain antibody is directed against IFN-gamma receptor. Said single domain antibody may be a *Camelidae* VHH.

Another aspect described herein is a method of treating an autoimmune disease or condition as cited herein, comprising administering to a patient an effective amount of an anti-TNF-alpha polypeptide further comprising a least one polypeptide selected from the group consisting of anti-IFN-gamma polypeptides, anti-TNF-alpha receptor polypeptide and anti-IFN-gamma receptor polypeptide, such polypeptides joined to each other as described below.

Such multi-specific constructs may have improved potency as inflammatory therapeutic compound over mono-specific constructs.

One aspect described herein is a composition comprising an anti-TNF-alpha polypeptide as disclosed herein and at least one polypeptide selected from the group consisting of anti-IFN-gamma polypeptide, anti-TNF-alpha receptor polypeptide and anti-IFN-gamma receptor polypeptide, for simultaneous, separate or sequential administration to a subject.

One aspect of the invention is use in a method for treating autoimmune disease comprising administering to an individual an effective amount of an anti-TNF-alpha polypeptide and a least one polypeptide selected from the group consisting of anti-IFN-gamma polypeptide, anti-TNF-alpha receptor polypeptide and anti-IFN-gamma receptor polypeptide, simultaneously, separately or sequentially.

Another aspect described herein is a kit containing an anti-TNF-alpha polypeptide and a least one polypeptide selected from the group consisting of anti-IFN-gamma polypeptide, anti-TNF-alpha receptor polypeptide and anti-IFN-gamma receptor polypeptide for simultaneous, separate or sequential administration to a subject. Also disclosed is that the kit may be used according to the invention. Also disclosed is that the kit may be used to treat the diseases as cited herein.

By simultaneous administration means the polypeptides are administered to a subject at the same time. For example, as a mixture of the polypeptides or a composition comprising said polypeptides. Examples include, but are not limited to a solution administered intraveneously, a tablet, liquid, topical cream, etc., wherein each preparation comprises the polypeptides of interest.

By separate administration means the polypeptides are administered to a subject at the same time or substantially the same time. The polypeptides are present in the kit as separate, unmixed preparations. For example, the different polypeptides may be present in the kit as individual tablets. The tablets may be administered to the subject by swallowing both tablets at the same time, or one tablet directly following the other.

By sequential administration means the polypeptides are administered to a subject sequentially. The polypeptides are present in the kit as separate, unmixed preparations. There is a time interval between doses. For example, one polypeptide might be administered up to 336, 312, 288, 264, 240, 216, 192, 168, 144, 120, 96, 72, 48, 24, 20, 16, 12, 8, 4, 2, 1, or 0.5 hours after the other component.

In sequential administration, one polypeptide may be administered once, or any number of times and in various doses before and/or after administration of another polypeptide. Sequential administration may be combined with simultaneous or sequential administration.

The medical uses of the anti-TNF-alpha polypeptide described below, also apply to the composition comprising an anti-TNF-alpha polypeptide as disclosed herein and at least one polypeptide selected from the group consisting of anti-IFN-gamma polypeptide, anti-TNF-alpha receptor polypeptide and anti-IFN-gamma receptor polypeptides, for simultaneous, separate or sequential administration to a subject as disclosed here above.

According to one aspect of the disclosure, an anti-IFN-gamma polypeptide anti-TNF-alpha a single domain antibody directed against IFN-gamma. Said single domain antibody may be a *Camelidae* VHH.

It is an embodiment of the disclosure that a single domain antibody directed against IFN-gamma corresponds to a sequence represented by any of SEQ ID NOs: 44 to 72 as shown in Table 6.

According to one aspect of the invention, anti-TNF-alpha a single domain antibody directed against TNF-alpha receptor. Said single domain antibody may be a *Camelidae* VHH.

According to one aspect of the disclosure, an anti-IFN-gamma receptor polypeptide anti-TNF-alpha a single domain antibody directed against IFN-gamma receptor. Said single domain antibody may be a *Camelidae* VHH.

In the present invention is an anti-TNF-alpha polypeptide as disclosed herein, the number of single domain antibodies directed against TNF-alpha is two or more. Such multivalent anti-TNF-alpha polypeptides have the advantage of unusually high functional affinity for the target, displaying much higher than expected inhibitory properties compared to their monovalent counterparts.

The multivalent anti-TNF-alpha polypeptides have functional affinities that are several orders of magnitude higher than the monovalent parent anti-TNF-alpha polypeptides. The inventors have found that the functional affinities of these multivalent polypeptides are much higher than those reported in the prior art for bivalent and multivalent antibodies. Surprisingly, anti-TNF-alpha polypeptides of the present invention linked to each other directly (SEQ ID No. 77 and 78) or via a short linker sequence show the high functional affinities expected theoretically with multivalent conventional four-chain antibodies.

The inventors have found that such large increased functional activities can be detected preferably with antigens composed of multidomain and multimeric proteins, either in straight binding assays or in functional assays, e.g. cytotoxicity assays.

Another embodiment of the present invention is an anti-TNF-alpha polypeptide as disclosed herein, wherein the number of single domain antibodies directed against TNF-alpha is two or more, said anti-TNF-alpha polypeptide comprising a sequence corresponding to any represented by SEQ ID NOs: 73 to 76.

The single domain antibodies may be joined to form any of the polypeptides disclosed herein comprising more than one single domain antibody using methods known in the art or any future method. For example, they may be fused by chemical cross-linking by reacting amino acid residues with an organic derivatising agent such as described by Blattler et al, Biochemistry 24,1517-1524; EP294703. Alternatively, the single domain antibody may be fused genetically at the DNA level *i.e.* a polynucleotide construct formed which encodes the complete polypeptide construct comprising one or more anti-target single domain antibodies and one or more anti-serum protein single domain antibodies. A method for producing bivalent or multivalent VHH polypeptide constructs is disclosed in PCT patent application WO 96/34103. One way of joining multiple single domain antibodies is via the genetic route by linking single domain antibody coding sequences either directly or *via* a peptide linker. For example, the C-terminal end of the first single domain antibody may be linked to the N-terminal end of the next single domain antibody. This linking mode can be extended in order to link additional single domain antibodies for the construction and production of tri-, tetra-, etc. functional constructs.

According to one aspect of the present invention, the single domain antibodies are linked to each other directly, without use of a linker. Contrary to joining bulky conventional antibodies where a linker sequence is needed to retain binding activity in the two subunits, polypeptides of the invention can be linked directly (SEQ ID No. 77 and 78) thereby avoiding potential problems of the linker sequence, such as antigenicity when administered to a human subject, instability of the linker sequence leading to dissociation of the subunits.
According to another aspect of the present invention, the single domain antibodies are linked to each other via a peptide linker sequence. Such linker sequence may be a naturally occurring sequence or a non-naturally occurring sequence. The linker sequence is expected to be non-immunogenic in the subject to which the anti-TNF-alpha polypeptide is administered. The linker sequence may provide sufficient flexibility to the multivalent anti-TNF-alpha polypeptide, at the same time being resistant to proteolytic degradation. A non-limiting example of a linker sequences is one that can be derived from the hinge region of VHHs described in WO 96/34103.

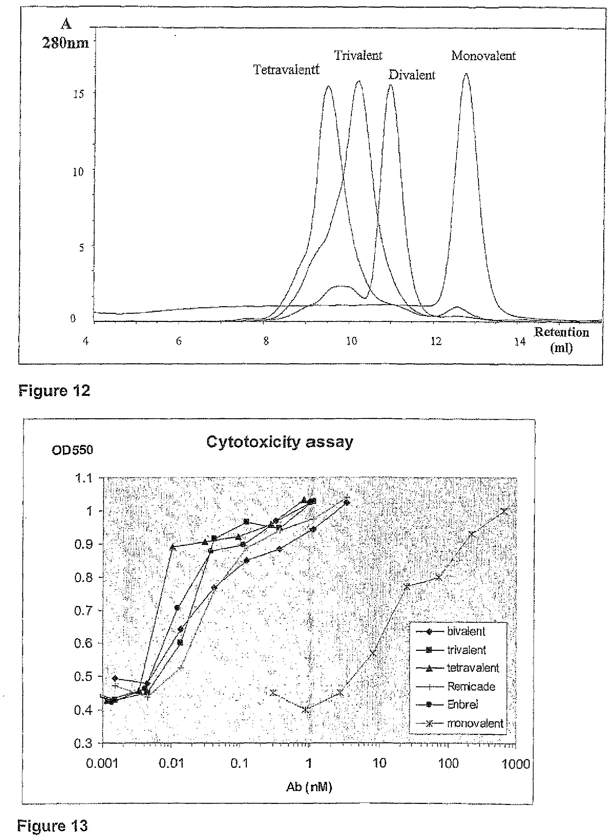

According to another aspect of the invention, multivalent single domain antibodies comprising more than two single domain antibodies can be linked to each other either directly or via a linker sequence. Such constructs are difficult to produce with conventional antibodies and due to steric hindrance of the bulky subunits, functionality will be lost or greatly diminished rather than increased considerably as seen with VHH's of the invention compared to the monovalent construct (see Figure 12 for gel filtration analyses of such multivalent VHH constructs).

The polypeptide constructs disclosed herein may be made by the skilled artisan according to methods known in the art or any future method. For example, VHHs may be obtained using methods known in the art such as by immunising a camel and obtaining hybridomas therefrom, or by cloning a library of single domain antibodies using molecular biology techniques known in the art and subsequent selection by using phage display.

According to an aspect of the invention an anti-TNF-alpha polypeptide may be a homologous sequence of a full-length anti-TNF-alpha polypeptide. According to another aspect of the invention, an anti-TNF-alpha polypeptide may be a functional portion of a full-length anti-TNF-alpha polypeptide. According to another aspect of the invention, an anti-TNF-alpha polypeptide may be a homologous sequence of a full-length anti-TNF-alpha polypeptide. According to another aspect of the invention, an anti-TNF-alpha polypeptide may be a functional portion of a homologous sequence of a full-length anti-TNF-alpha polypeptide. According to an aspect of the invention an anti-TNF-alpha polypeptide may comprise a sequence of an anti-TNF-alpha polypeptide.

According to an aspect of the invention a single domain antibody used to form an anti-TNF-alpha polypeptide may be a complete single domain antibody (*e.g.* a VHH) or a homologous sequence thereof. According to another aspect of the invention, a single domain antibody used to form the polypeptide construct may be a functional portion of a complete single domain antibody. According to another aspect of the invention, a single domain antibody used to form the polypeptide construct may be a homologous sequence of a complete single domain antibody. According to another aspect of the invention, a single domain antibody used to form the polypeptide construct may be a functional portion of a homologous sequence of a complete single domain antibody.

As used herein, an homologous sequence of the present invention may comprise additions, deletions or substitutions of one or more amino acids, which do not substantially alter the functional characteristics of the polypeptides of the invention. The number of amino acid deletions or substitutions is preferably up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39,40, 1,42, 43, 44, 5, 46, 7, 48, 49, 50, 1, 52, 53, 54, 55, 56, 57, 58, 59, 60, 61, 62, 63, 64, 65, 66, 67, 68, 69 or 70 amino acids.

A homologous sequence according to the present invention may a polypeptide modified by the addition, deletion or substitution of amino acids, said modification not substantially altering the functional characteristics compared with the unmodified polypeptide.

A homologous sequence according to the present invention may be a polypeptide modified by the addition, deletion or substitution of amino acids, said modification not substantially altering the functional characteristics compared with the unmodified polypeptide.

A homologous sequence according to the present invention may be a sequence which exists in other *Camelidae* species such as, for example, camel, dromedary, Ilama, alpaca, guanaco etc.

Where homologous sequence indicates sequence identity, it means a sequence which presents a high sequence identity (more than 70%, 75%, 80%, 85%, 90%, 95% or 98% sequence identity) with the parent sequence and is preferably characterised by similar properties of the parent sequence, namely affinity, said identity calculated using known methods.

Alternatively, an homologous sequence may also be any amino acid sequence resulting from allowed substitutions at any number of positions of the parent sequence according to the formula below:
Ser substituted by Ser, Thr, Gly, and Asn;
Arg substituted by one of Arg, His, Gin, Lys, and Glu;
Leu substituted by one of Leu, Ile, Phe, Tyr, Met, and Val;
Pro substituted by one of Pro, Gly, Ala, and Thr;
Thr substituted by one of Thr, Pro, Ser, Ala, Gly, His, and Gin;
Ala substituted by one of Ala, Gly, Thr, and Pro;
Val substituted by one of Val, Met, Tyr, Phe, Ile, and Leu;
Gly substituted by one of Gly, Ala, Thr, Pro, and Ser;
Ile substituted by one of Ile, Met, Tyr, Phe, Val, and Leu;
Phe substituted by one of Phe, Trp, Met, Tyr, lie, Val, and Leu;
Tyr substituted by one of Tyr, Trp, Met, Phe, Ile, Val, and Leu;
His substituted by one of His, Glu, Lys, Gin, Thr, and Arg;
Gln substituted by one of Gin, Glu, Lys, Asn, His, Thr, and Arg;
Asn substituted by one of Asn, Glu, Asp, Gin, and Ser;
Lys substituted by one of Lys, Glu, Gln, His, and Arg;
Asp substituted by one of Asp, Glu, and Asn;
Glu substituted by one of Glu, Asp, Lys, Asn, Gln, His, and Arg;
Met substituted by one of Met, Phe, Ile, Val, Leu, and Tyr.

A homologous nucleotide sequence according to the present invention may refer to nucleotide sequences of more than 50, 100, 200, 300, 400, 500, 600, 800 or 1000 nucleotides able to hybridize to the reverse-complement of the nucleotide sequence capable of encoding the patent sequence, under stringent hybridisation conditions (such as the ones described by Sambrook et al., Molecular Cloning, Laboratory Manuel, Cold Spring, Harbor Laboratory press, New York).

As used herein, a functional portion refers to a sequence of a single domain antibody that is of sufficient size such that the interaction of interest is maintained with affinity of 1 x 10⁻⁶ M or better.

Alternatively, a functional portion comprises a partial deletion of the complete amino acid sequence and still maintains the binding site(s) and protein domain(s) necessary for the binding of and interaction with the target.

As used herein, a functional portion refers to less than 100% of the complete sequence (*e*.*g*., 99%, 90%, 80%, 70%, 60% 50%, 40%, 30%, 20%, 10%, 5%, 1% etc.), but comprises 5 or more amino acids or 15 or more nucleotides.

Targets as mentioned herein such as TNF-alpha, TNF-alpha receptor, serum proteins (*e.g*. serum albumin, serum immunoglobulins, thyroxine-binding protein, transferrin, fibrinogen) and IFN-gamma, IFN-gamma receptor may be fragments of said targets. Thus a target is also a fragment of said target, capable of eliciting an immune response. A target is also a fragment of said target, capable of binding to a single domain antibody raised against the full length target.

A fragment as used herein refers to less than 100% of the sequence (e.g., 99%, 90%, 80%, 70%, 60%, 50%, 40%, 30%, 20%, 10% etc.), but comprising 5, 6, 7, 8, 9, 10, 12, 13, 14, 15, 16, 17, 18, 19, 20, 21, 22, 23, 24, 25 or more amino acids. A fragment is of sufficient length such that the interaction of interest is maintained with affinity of 1 x 10-6 M or better.

A fragment as used herein also refers to optional insertions, deletions and substitutions of one or more amino acids which do not substantially alter the ability of the target to bind to a single domain antibody raised against the wild-type target. The number of amino acid insertions deletions or substitutions is preferably up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 amino acids.

A homologous sequence of the present invention may include an anti-TNF-alpha polypeptide which has been humanised. The humanisation of antibodies of the new class of VHHs would further reduce the possibility of unwanted immunological reaction in a human individual upon administration.

One embodiment of the present invention relates to a method for preparing modified polypeptides based upon llama antibodies by determining the amino acid residues of the antibody variable domain (VHH) which may be modified without diminishing the native affinity of the domain for antigen and while reducing its immunogenicity with respect to a heterologous species; the use of VHHs having modifications at the identified residues which are useful for administration to heterologous species; and to the VHH so modified.

More specifically, the invention relates to the preparation of modified VHHs, which are modified for administration to humans, the resulting VHH themselves, and the use of such "humanized" VHHs in the treatment of diseases in humans. By humanised is meant mutated so that immunogenicity upon administration in human patients is minor or nonexistent. Humanising a polypeptide, according to the present invention, comprises a step of replacing one or more of the *Camelidae* amino acids by their human counterpart as found in the human consensus sequence, without that polypeptide losing its typical character, *i.e,* the humanisation does not significantly affect the antigen binding capacity of the resulting polypeptide. Such methods are known by the skilled addressee.

Humanization of *Camelidae* single domain antibodies requires the introduction and mutagenesis of a limited amount of amino acids in a single polypeptide chain. This is in contrast to humanization of scFv, Fab, (Fab)2 and IgG, which requires the introduction of amino acid changes in two chains, the light and the heavy chain and the preservation of the assembly of both chains.

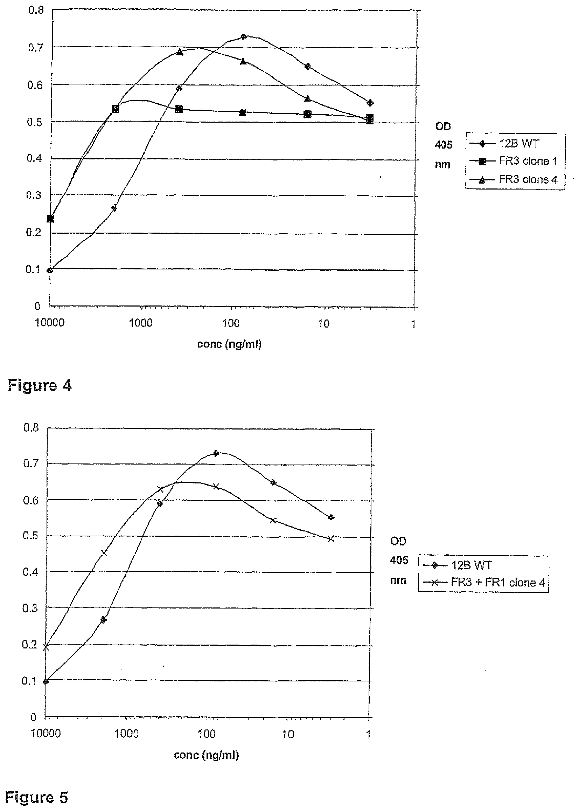

As a non-limited example, the polypeptide of VHH#12B containing human-like residues in FR2 was humanized. Humanization required mutagenesis of residues in FR1 at position 1 and 5 which were introduced by the primer used for repertoire cloning and do not occur naturally in the llama sequence. Mutagenesis of those residues did not result in loss of binding and/or inhibition activity. Humanization also required mutagenesis of residues in FR3 at position 74, 76, 83, 84, 93. Mutagenesis of those residues did not result in a dramatic loss of binding and/or inhibition activity (see Figure 4). Combining the mutations of FR1 and FR3 therefore did not affect the binding and/or inhibition activity (Figure 5). Humanization also required mutagenesis of residues In FR4 at position 108. Mutagenesis of Q108L resulted in lower production level in *Escherichia coli.* Position 108 is solvent exposed in camelid VHH, while in human antibodies this position is buried at the VH-VL interface (Spinelli, 1996; Nieba, 1997). In isolated VHs position 108 is solvent exposed. The introduction of a non-polar hydrophobic Leu instead of polar uncharged Gin can have a drastic effect on the intrinsic folding/stability of the molecule.

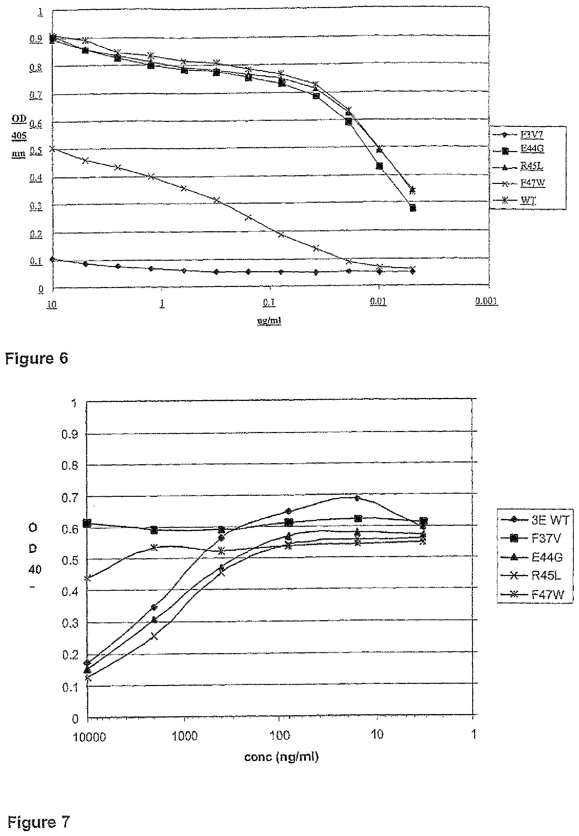

As a non-limited example, the polypeptide represented in the VHH#3E containing camelid hallmark residues at position 37, 44, 45 and 47 with hydrophilic characteristics, was humanized. Replacement of the hydrophilic residues by human hydrophobic residues at positions 44 and 45 (E44G and R45L), did not have an effect on binding and/or inhibition. However, loss of binding and/or inhibition activity was observed when F37V and F47W were introduced. Modeling data confirmed the critical residue 37 to preserve the integrity of the CDR3 loop conformation and hence on activity (see Figure 6)(all numbering according to the Kabat).

SEQ ID NO: 3 and 14 display more than 90% amino acid sequence homology to human VH framework regions and therefore said VHH might be administered to patients directly without expectation of an immune response therefrom, and without the additional burden of humanisation. Therefore, one aspect described herein allows for the direct administration of the polypeptide comprising SEQ ID NO: 3 and 14, homologous sequence thereof, or a functional portion of an homologous sequence thereof to a patient in need of the same.

One embodiment of the present invention is a method for humanizing a VHH comprising the steps of replacing of any of the following residues either alone or in combination:
FR1 position 1, 5, 28 and 30,
the hallmark amino acid at position 44 and 45 in FR2,
FR3 residues 74, 75, 76, 83, 84, 93 and 94 ,
and positions 103, 104, 108 and 111 in FR4;
numbering according to the Kabat numbering.

One embodiment of the present invention is an anti-TNF-alpha polypeptide, or a nucleic acid capable of encoding said polypeptide for use in a method for treating, preventing and/or alleviating the symptoms of disorders relating to inflammatory processes. TNF-alpha is involved in inflammatory processes, and the blocking of TNF-alpha action can have an antiInflammatory effect, which is highly desirable in certain disease states such as, for example, Crohn's disease. Our Examples demonstrate VHHs according to the invention which bind TNF-alpha and moreover, block its binding to the TNF-alpha receptor.

The anti-TNF-alpha polypeptides of the present invention are applicable to autoimmune diseases, such as Addison's disease (adrenal), Autoimmune diseases of the ear (ear), Autoimmune diseases of the eye (eye), Autoimmune hepatitis (liver), Autoimmune parotitis (parotid glands), Crohn's disease (intestine), Diabetes Type I (pancreas), Epididymitis (epididymis), Glomerulonephritis (kidneys), Graves' disease (thyroid), Guillain-Barre syndrome (nerve cells), Hashimoto's disease (thyroid), Hemolytic anemia (red blood cells), Systemic lupus erythematosus (multiple tissues), Male infertility (sperm), Multiple sclerosis (nerve cells), Myasthenia Gravis (neuromuscular junction), Pemphigus (primarily skin), Psoriasis (skin), Rheumatic fever (heart and joints), Rheumatoid arthritis (joint lining), Sarcoidosis (multiple tissues and organs), Scieroderma (skin and connective tissues), Sjogren's syndrome (exocrine glands, and other tissues), Spondyloarthropathies (axial skeleton, and other tissues), Thyroiditis (thyroid), Vasculitis (blood vessels). Within parenthesis is the tissue affected by the disease. This listing of autoimmune diseases is intended to be exemplary rather than inclusive.

Autoimmune conditions for which the anti-TNF-alpha polypeptides of the present invention is applicable include, for example, AIDS, atopic allergy, bronchial asthma, eczema, leprosy, schizophrenia, inherited depression, transplantation of tissues and organs, chronic fatigue syndrome, Alzheimer's disease, Parkinson's disease, myocardial infarction, stroke, autism, epilepsy, Arthus's phenomenon, anaphylaxis, and alcohol and drug addiction. In the above-identified autoimmune conditions, the tissue affected is the primary target, in other cases it is the secondary target. These conditions are partly or mostly autoimmune syndromes. Therefore, in treating them, it is possible to use the same methods, or aspects of the same methods that are herein disclosed, sometimes in combination with other methods.

Another embodiment of the present invention is a use of an anti-TNF-alpha polypeptide according to the invention, or a nucleic acid capable of encoding said polypeptide for the preparation of a medicament for treating a disorder relating to inflammatory processes. Examples of disorders include rheumatoid arthritis, Crohn's disease, ulcerative colitis, inflammatory bowel syndrome and multiple sclerosis

Polypeptides and nucleic acids according to the present invention may be used in a method of administering to a subject by conventional routes, such as intravenously. However, a special property of the anti-TNF-alpha polypeptides of the invention is that they penetrate barriers such as tissue membranes and/or tumours and act locally and act locally thereon, and they are sufficiently stable to withstand extreme environments such as in the stomach. Therefore, another aspect of the present invention relates to the delivery of anti-TNF-alpha polypeptides.

A subject according to the invention can be any mammal susceptible to treatment by therapeutic polypeptides.

Oral delivery of anti-TNF-alpha polypeptides of the invention results in the provision of such molecules In an active form in the colon at local sites that are affected by the disorder. These sites may be highly inflamed and contain TNF-alpha-producing cells. The anti-TNI-alpha polypeptides of the invention which bind to TNF-alpha can neutralise the TNF-alpha locally, avoiding distribution throughout the whole body and thus limiting negative side-effects. Genetically modified microorganisms such as *Micrococcus lactis* are able to secrete antibody or functional portions thereof. Such modified microorganisms can be used as vehicles for local production and delivery of antibodies or functional portions thereof in the intestine. By using a strain which produces an anti-TNF-alpha polypeptide, inflammatory bowel syndrome could be treated.

Another aspect of the invention involves delivering anti-TNF polypeptides by using surface expression on or secretion from non-invasive bacteria, such as Gram-positive host organisms like *Lactococcus spec.* using a vector such as described in WO00/23471.

One embodiment of the present invention is an anti-TNF-alpha polypeptides as disclosed herein for use in a method of treating, preventing and/or alleviating the symptoms of disorders susceptible to modulation by a TNF-alpha modulating substance which is able pass through the gastric environment without the substance being inactivated.

Examples of disorders are any that cause inflammation, including, but not limited to rheumatoid arthritis, Crohn's disease, ulcerative colitis, inflammatory bowl syndrome, and multiple sclerosis. As known by persons skilled in the art, once in possession of said polypeptide construct, formulation technology may be applied to release a maximum amount of polypeptide in the right location (in the stomach, in the colon, etc.). This method of delivery is important for treating, prevent and/or alleviate the symptoms of disorders whose targets are located in the gut system.

An aspect of the invention is used in a method for treating, preventing and/or alleviating the symptoms of a disorder susceptible to modulation by a TNF-alpha modulating substance which is able pass through the gastric environment without being inactivated, by orally administering to a subject an anti-TNF-akpha polypeptide as disclosed herein.

Another embodiment of the present invention is a use of an anti-TNF-alpha polypeptide as disclosed herein for the preparation of a medicament for treating, preventing and/or alleviating the symptoms of disorders susceptible to modulation by a TNF-alpha modulating substance which is able pass through the gastric environment without being inactivated.

An aspect of the invention is used in a method for delivering a TNF-alpha modulating substance to the gut system without said substance being inactivated, by orally administering to a subject an anti-TNF-alpha polypeptide as disclosed herein.

An aspect of the invention is used in a method for delivering a TNF-alpha modulating substance to the bloodstream of a subject without the substance being inactivated, by orally administering to a subject an anti-TNF-alpha polypeptide as disclosed herein.

Another embodiment of the present invention is an anti-TNF-alpha polypeptide as disclosed herein for use in a method of treating, preventing and/or alleviating the symptoms or disorders susceptible to modulation by a TNF-alpha modulating substance delivered to the vaginal and/or rectal tract.

Examples of disorders are any that cause inflammation, including, but not limited to rheumatoid arthritis, Crohn's disease, ulcerative colitis, inflammatory bowl syndrome, and multiple sclerosis. In a non-limiting example, a formulation according to the invention comprises an anti-TNF-alpha polypeptides as disclosed herein, in the form of a gel, cream, suppository, film, or in the form of a sponge or as a vaginal ring that slowly releases the active ingredient over time (such formulations are described in EP 707473, EP 684814, US 5629001).

An aspect of the invention is used in a method for treating, preventing and/or alleviating the symptoms of disorders susceptible to modulation by a TNF-alpha modulating substance delivered to the vaginal and/or rectal tract, by vaginally and/or rectally administering to a subject an anti-TNF-alpha polypeptide as disclosed herein.

Another embodiment of the present invention is a use of an anti-TNF-alpha polypeptide as disclosed herein for the preparation of a medicament for treating, preventing and/or alleviating the symptoms of disorders susceptible to modulation by a TNF-alpha modulating substance delivered to the vaginal and/or rectal tract.

An aspect of the invention is used in a method for delivering a TNF-alpha modulating substance to the vaginal and/or rectal tract without being said substance being inactivated, by administering to the vaginal and/or rectal tract of a subject an anti-TNF-alpha polypeptide as disclosed herein.

An aspect of the invention is used in a method for delivering a TNF-alpha modulating substance to the bloodstream of a subject without said substance being inactivated, by administering to the vaginal and/or rectal tract of a subject an anti-TNF-alpha polypeptide as disclosed herein.

Another embodiment of the present invention is an anti-TNF-alpha polypeptide as disclosed herein, for use in a method of treating, preventing and/or alleviating the symptoms of disorders susceptible to modulation by a TNF-alpha modulating substance delivered to the nose, upper respiratory tract and/or lung.

Examples of disorders are any that cause inflammation, including, but not limited to rheumatoid arthritis, Crohn's disease, ulcerative colitis, inflammatory bowl syndrome, and multiple sclerosis. In a non-limiting example, a formulation according to the invention, comprises an anti-TNF-alpha polypeptide as disclosed herein in the form of a nasal spray (*e.g.* an aerosol) or inhaler. Since the polypeptide construct is small, it can reach its target much more effectively than therapeutic IgG molecules.

An aspect of the invention is use in a method for treating, preventing and/or alleviating the symptoms of disorders susceptible to modulation by a TNF-alpha modulating substance delivered to the upper respiratory tract and lung, by administering to a subject an anti-TNF-alpha polypeptide as disclosed herein, by inhalation through the mouth or nose.

Another embodiment of the present Invention is a use of an anti-TNF-alpha polypeptide as disclosed herein for the preparation of a medicament for treating, preventing and/or alleviating the symptoms of disorders susceptible to modulation by a TNF-alpha modulating substance delivered to the nose, upper respiratory tract and/or lung, without said polypeptide being inactivated.

An aspect of the invention is use in a method for delivering a TNF-alpha modulating substance to the nose, upper respiratory tract and lung without inactivation, by administering to the nose, upper respiratory tract and/or lung of a subject an anti-TNF-alpha polypeptide as disclosed herein.

An aspect of the invention is use in a method for delivering a TNF-alpha modulating substance to the bloodstream of a subject without inactivation by administering to the nose, upper respiratory tract and/or lung of a subject an anti-TNF-alpha polypeptide as disclosed herein.

One embodiment of the present invention is an anti-TNF-alpha polypeptide as disclosed herein for use in a method of treating, preventing and/or alleviating the symptoms of disorders susceptible to modulation by a TNF-alpha modulating substance delivered to the intestinal mucosa, wherein said disorder increases the permeability of the intestinal mucosa. Because of their small size, an anti-TNF-alpha polypeptide as disclosed herein can pass through the intestinal mucosa and reach the bloodstream more efficiently in subjects suffering from disorders which cause an increase in the permeability of the intestinal mucosa, for example Crohn's disease.

An aspect of the invention is use in a method for treating, preventing and/or alleviating the symptoms of disorders susceptible to modulation by a TNF-alpha modulating substance delivered to the intestinal mucosa, wherein said disorder increases the permeability of the intestinal mucosa, by orally administering to a subject an anti-TNF-alpha polypeptide as disclosed herein.

This process can be even further enhanced by an additional aspect of the present invention - the use of active transport carriers. In this aspect of the invention, VHH is fused to a carrier that enhances the transfer through the intestinal wall into the bloodstream. In a non-limiting example, this "carrier" is a second VHH which is fused to the therapeutic VHH. Such fusion constructs are made using methods known in the art. The "carrier" VHH binds specifically to a receptor on the intestinal wall which induces an active transfer through the wall.

Another embodiment of the present invention is a use of an anti-TNF-alpha polypeptide as disclosed herein for the preparation of a medicament for treating, preventing and/or alleviating the symptoms of disorders susceptible to modulation by a TNF-alpha, modulating substance delivered to the intestinal mucosa, wherein said disorder increases the permeability of the intestinal mucosa.

An aspect of the invention is use in a method for delivering a TNF-alpha modulating substance to the intestinal mucosa without being inactivated, by administering orally to a subject an anti-TNF-alpha polypeptide of the invention.

An aspect of the invention is use in a method for delivering a TNF-alpha modulating substance to the bloodstream of a subject without being inactivated, by administering orally to a subject an anti-TNF-alpha polypeptide of the invention.

This process can be even further enhanced by an additional aspect of the present invention - the use of active transport carriers. In this aspect of the invention, an anti-TNF-alpha polypeptide as described herein Is fused to a carrier that enhances the transfer through the intestinal wall into the bloodstream. In a non-limiting example, this "carrier" is a VHH which is fused to said polypeptide. Such fusion constructs made using methods known in the art. The "carrier" VHH binds specifically to a receptor on the intestinal wall which induces an active transfer through the wall.

One embodiment of the present invention is an anti-TNF-alpha polypeptide as disclosed herein for use in a method of treating, preventing and/or alleviating the symptoms of disorders susceptible to modulation by a TNF-alpha modulating substance which is able pass through the tissues beneath the tongue effectively.

Examples of disorders are any that cause inflammation, including, but not limited to rheumatoid arthritis, Crohn's disease, ulcerative colitis, inflammatory bowl syndrome, and multiple sclerosis. A formulation of said polypeptide construct as disclosed herein, for example, a tablet, spray, drop is placed under the tongue and adsorbed through the mucus membranes into the capillary network under the tongue.

An aspect of the invention is use in a method for treating, preventing and/or alleviating the symptoms of disorders susceptible to modulation by a TNF-alpha modulating substance which is able pass through the tissues beneath the tongue effectively, by sublingually administering to a subject an anti-TNF-alpha polypeptide as disclosed herein.

Another embodiment of the present invention is a use of an anti-TNF-alpha polypeptide as disclosed herein for the preparation of a medicament for treating, preventing and/or alleviating the symptoms of disorders susceptible to modulation by a TNF-alpha modulating substance which is able to pass through the tissues beneath the tongue.

An aspect of the invention is use in a method for delivering a TNF-alpha modulating substance to the tissues beneath the tongue without being inactivated, by administering sublingually to a subject an anti-TNF-alpha polypeptide as disclosed herein.

An aspect of the invention is use in a method for delivering a TNF-alpha modulating substance to the bloodstream of a subject without being inactivated, by administering orally to a subject an anti-TNF-alpha polypeptide as disclosed herein.

One embodiment of the present invention is an anti-TNF-alpha polypeptide as disclosed herein for use in a method of treating, preventing and/or alleviating the symptoms of disorders susceptible to modulation by a TNF-alpha modulating substance which is able pass through the skin effectively.

Examples of disorders are any that cause inflammation, induing, but not limited to rheumatoid arthritis, Crohn's disease, ulcerative colitis, inflammatory bowl syndrome, and multiple sclerosis. A formulation of said polypeptide construct, for example, a cream, film, spray, drop, patch, is placed on the skin and passes through.

An aspect of the invention is use in a method for treating, preventing and/or alleviating the symptoms of disorders susceptible to modulation by a TNF-alpha modulating substance which is able pass through the skin effectively, by topically administering to a subject an anti-TNF-alpha polypeptide as disclosed herein.

Another embodiment of the present invention is a use of an anti-TNF-alpha polypeptide as disclosed herein for the preparation of a medicament for treating, preventing and/or alleviating the symptoms of disorders susceptible to modulation by a TNF-alpha modulating substance which is able pass through the skin effectively.

An aspect of the invention is use in a method for delivering a TNF-alpha modulating substance to the skin without being inactivated, by administering topically to a subject an anti-TNF-alpha polypeptide as disclosed herein.

An aspect of the invention is use in a method for delivering a TNF-alpha modulating substance to the bloodstream of a subject, by administering topically to a subject an anti-TNF-alpha polypeptide as disclosed herein.

In another embodiment of the present invention, an anti-TNF-alpha polypeptide further comprises a carrier single domain antibody (e.g. VHH) which acts as an active transport carrier for transport said anti-TNF-alpha polypeptides, from the lung lumen to the blood.

An anti-TNF-alpha polypeptide further comprising a carrier binds specifically to a receptor present on the mucosal surface (bronchial epithelial cells) resulting in the active transport of the polypeptides from the lung lumen to the blood. The carrier single domain antibody may be fused to the polypeptide construct. Such fusion constructs may be made using methods known in the art and are describe herein. The "carrier" single domain antibody binds specifically to a receptor on the mucosal surface which induces an active transfer through the surface.

Another aspect described herein is a method to determine which single domain antibodies (e.g. VHHs) are actively transported into the bloodstream upon nasal administration. Similarly, a naïve or immune VHH phage library can be administered nasally, and after different time points after administration, blood or organs can be isolated to rescue phages that have been actively transported to the bloodstream. A non-limiting example of a receptor for active transport from the lung lumen to the bloodstream is the Fc receptor N (FcRn). One aspect described herein includes the VHH molecules identified by the method. Such VHH can then be used as a carrier VHH for the delivery of a therapeutic VHH to the corresponding target in the bloodstream upon nasal administration.

In one aspect of the invention, one can use an anti-TNF-alpha polypeptide as disclosed herein, in order to screen for agents that modulate the binding of the polypeptide to TNF-alpha. When identified in an assay that measures binding or said polypeptide displacement alone, agents will have to be subjected to functional testing to determine whether they would modulate the action of the antigen *in vivo.* Examples of screening assays are given below primarily in respect of SEQ ID NO: 3, though any anfi-TNF-alpha polypeptide as disclosed herein as disclosed herein may be appropriate.

In an example of a displacement experiment, phage or cells expressing TNF-alpha or a fragment thereof are incubated in binding buffer with, for example, a polypeptide represented by SEQ ID NO: 3 which has been labeled, in the presence or absence of increasing concentrations of a candidate modulator. To validate and calibrate the assay, control competition reactions using increasing concentrations of said polypeptide and which is unlabeled, can be performed. After incubation, cells are washed extensively, and bound, labeled polypeptide is measured as appropriate for the given label *(e.g.,* scintillation counting, fluorescence, etc.). A decrease of at least 10% in the amount of labeled polypeptide bound in the presence of candidate modulator indicates displacement of binding by the candidate modulator. Candidate modulators are considered to bind specifically in this or other assays described herein if they displace 50% of labeled polypeptide (sub-saturating polypeptide dose) at a concentration of 1 µM or less.

Alternatively, binding or displacement of binding can be monitored by surface plasmon resonance (SPR). Surface plasmon resonance assays can be used as a quantitative method to measure binding between two molecules by the change in mass near an immobilized sensor caused by the binding or loss of binding of, for example, the polypeptide represented by SEQ ID NO: 3 from the aqueous phase to TNF-alpha immobilized in a membrane on the sensor. This change in mass is measured as resonance units versus time after injection or removal of the said polypeptide or candidate modulator and is measured using a Biacore Biosensor (Biacore AB). TNF-alpha can be for example immobilized on a sensor chip (for example, research grade CM5 chip; Biacore AB) in a thin film lipid membrane according to methods described by Salamon et al. (Salamon et al., 1996, Biophys J. 71: 283-294; Salamon et al., 2001, Biophys. J. 80: 1557-1567; Salamon et al., 1999, Trends Biochem. Sci. 24: 213-219). Sarrio *et al.* demonstrated that SPR can be used to detect ligand binding to the GPCR A(1) adenosine receptor immobilized in a lipid layer on the chip (Sarrio et al., 2000, Moi. Cell. Biol. 20: 5164-5174). Conditions for the binding of SEQ ID NO:3 to TNF-alpha in an SPR assay can be fine-tuned by one of skill in the art using the conditions reported by Sarrio *et al.* as a starting point.

SPR can assay for modulators of binding in at least two ways. First, a polypeptide represented by SEQ ID NO: 3, for example, can be pre-bound to immobilized TNF-alpha followed by Injection of candidate modulator at a concentration ranging from 0.1 nM to 1 µM. Displacement of the bound polypeptide can be quantitated, permitting detection of modulator binding. Alternatively, the membrane-bound TNF-alpha can be pre-incubated with a candidate modulator and challenged with, for example, a polypeptide represented by SEQ ID NO: 3. A difference in binding affinity between said polypeptide and TNF-alpha pre-incubated with the modulator, compared with that between said polypeptide and TNF-alpha in absence of the modulator will demonstrate binding or displacement of said polypeptide in the presence of modulator. In either assay, a decrease of 10% or more in the amount of said polypeptide bound in the presence of candidate modulator, relative to the amount of said polypeptides bound in the absence of candidate modulator indicates that the candidate modulator inhibits the interaction of TNF-alpha and said polypeptide.

Another method of detecting inhibition of binding of, for example, a polypeptide represented by SEQ ID NO: 3, to TNF-alpha uses fluorescence resonance energy transfer (FRET). FRET is a quantum mechanical phenomenon that occurs between a fluorescence donor (D) and a fluorescence acceptor (A) in close proximity to each other (usually < 100 A of separation) if the emission spectrum of D overlaps with the excitation spectrum of A. The molecules to be tested, *e.g.* a polypeptide represented by SEQ ID NO: 3 and a TNF-alpha are labelled with a complementary pair of donor and acceptor fluorophores. While bound closely together by the TNF-alpha: polypeptide interaction, the fluorescence emitted upon excitation of the donor fluorophore will have a different wavelength from that emitted in response to that excitation wavelength when the said polypeptide and TNF-alpha are not bound, providing for quantitation of bound versus unbound molecules by measurement of emission intensity at each wavelength. Donor fluorophores with which to label the TNF-alpha are well known in the art. Of particular interest are variants of the A. Victoria GFP known as Cyan FP (CFP, Donor (D)) and Yellow FP (YFP, Acceptor (A)). As an example, the YFP variant can be made as a fusion protein with TNF-alpha. Vectors for the expression of GFP variants as fusions (Clontech) as well as fluorophore-labeled reagents (Molecular Probes) are known in the art. The addition of a candidate modulator to the mixture of fluorescentlly-labelled polypeptide and YFP-TNF-alpha will result in an inhibition of energy transfer evidenced by, for example, a decrease in YFP fluorescence relative to a sample without the candidate modulator. In an assay using FRET for the detection of TNF-alpha: polypeptide interaction, a 10% or greater decrease in the intensity of fluorescent emission at the acceptor wavelength in samples containing a candidate modulator, relative to samples without the candidate modulator, indicates that the candidate modulator inhibits the TNF-alpha:polypeptide interaction.

A sample as used herein may be any biological sample containing TNF-alpha such as clinical (*e.g*. cell fractions, whole blood, plasma, serum, tissue, cells, etc.), derived from clinical, agricultural, forensic, research, or other possible samples. The clinical samples may be from human or animal origin. The sample analysed can be both solid or liquid in nature. It is evident when solid materials are used, these are first dissolved in a suitable solution.

A variation on FRET uses fluorescence quenching to monitor molecular interactions. One molecule in the interacting pair can be labelled with a fluorophore, and the other with a molecule that quenches the fluorescence of the fluorophore when brought into close apposition with it. A change in fluorescence upon excitation is indicative of a change in the association of the molecules tagged with the fluorophore:quencher pair. Generally, an increase in fluorescence of the labelled TNF-alpha is indicative that anti-TNF-alpha polypeptide bearing the quencher has been displaced. For quenching assays, a 10% or greater increase in the intensity of fluorescent emission in samples containing a candidate modulator, relative to samples without the candidate modulator, indicates that the candidate modulator inhibits TNF-alpha: anti-TNF-alpha polypeptide interaction.

In addition to the surface plasmon resonance and FRET methods, fluorescence polarization measurement is useful to quantitate binding. The fluorescence polarization value for a fluorescently-tagged molecule depends on the rotational correlation time or tumbling rate. Complexes, such as those formed by TNF-alpha associating with a fluorescently labelled anti-TNF-alpha polypeptide, have higher polarization values than uncomplexed, labelled polypeptide. The inclusion of a candidate inhibitor of the TNF-alpha:anti-TNF-alpha polypeptide interaction results in a decrease in fluorescence polarization, relative to a mixture without the candidate inhibitor, if the candidate inhibitor disrupts or inhibits the interaction of TNF-alpha with said polypeptide. Fluorescence polarization is well suited for the identification of small molecules that disrupt the formation of TNF-alpha:anti-TNF-alpha polypeptide complexes. A decrease of 10% or more in fluorescence polarization in samples containing a candidate modulator, relative to fluorescence polarization in a sample lacking the candidate modulator, indicates that the candidate modulator inhibits the TNF-alpha : anti-TNF-alpha polypeptide interaction.

Another alternative for monitoring TNF-alpha : anti-TNF-alpha polypeptide interactions uses a biosensor assay. ICS biosensors have been described in the art (Australian Membrane Biotechnology Research Institute; Cornell B, Braach-Maksvytis V, King L, Osman P, Raguse B, Wieczorek L, and Pace R. "A biosensor that uses ion-channel switches" Nature 1997, 387, 580). In this technology, the association of TNF-alpha and a anti-TNF-alpha polypeptide is coupled to the closing of gramacidin-fiacilitated ion channels in suspended membrane bilayers and thus to a measurable change in the admittance (similar to impedence) of the biosensor. This approach is linear over six orders of magnitude of admittance change and is ideally suited for large scale, high throughput screening of small molecule combinatorial libraries. A 10% or greater change (increase or decrease) in admittance in a sample containing a candidate modulator, relative to the admittance of a sample lacking the candidate modulator, indicates that the candidate modulator inhibits the interaction of TNF-alpha and said polypeptide. It is important to note that in assays testing the interaction of TNF-alpha with an anti-TNF-alpha polypeptide, it is possible that a modulator of the interaction need not necessarily interact directly with the domain(s) of the proteins that physically interact with said polypeptide. It is also possible that a modulator will interact at a location removed from the site of interaction and cause, for example, a conformational change in the TNF-alpha. Modulators (inhibitors or agonists) that act in this manner are nonetheless of interest as agents to modulate the binding of TNF-alpha to its receptor.

Any of the binding assays described can be used to determine the presence of an agent in a sample, e.g., a tissue sample, that binds to TNF-alpha, or that affects the binding of, for example, a polypeptide represented by SEQ ID NO: 3 to the TNF-alpha. To do so a TNF-alpha is reacted with said polypeptide in the presence or absence of the sample, and polypeptide binding is measured as appropriate for the binding assay being used. A decrease of 10% or more in the binding of said polypeptide indicates that the sample contains an agent that modulates the binding of said polypeptide to the TNF-alpha, Of course, the above-generalized method might easily be applied to screening for candidate modulators which alter the binding between any anti-TNF-alpha polypeptide of the invention, an homologous sequence thereof, a functional portion thereof or a functional portion of an homologous sequence thereof, and TNF-alpha or a fragment thereof.

One embodiment described herein is an unknown agent identified by the method disclosed herein.

One embodiment described herein is an unknown agent identified by the method disclosed herein for use in treating, preventing and/or alleviating the symptoms of disorders relating to inflammatory processes.

Another embodiment described herein is a use of an unknown agent identified by the method disclosed herein for use in treating, preventing and/or alleviating the symptoms of disorders relating to inflammatory processes.

Examples of disorders include rheumatoid arthritis, Crohn's disease, ulcerative colitis, inflammatory bowel syndrome and multiple sclerosis

A cell that is useful according to the invention is preferably selected from the group consisting of bacterial cells such as, for example, *E*. *coli,* yeast cells such as, for example, *S. cerevisiae, P*. *pastoris,* insect cells or mammal cells.

A cell that is useful according to the invention can be any cell into which a nucleic acid sequence encoding a polypeptide comprising an anti-TNF-alpha of the invention, an homologous sequence thereof, a functional portion thereof or a functional portion of an homologous sequence thereof according to the invention can be introduced such that the polypeptide is expressed at natural levels or above natural levels, as defined herein. Preferably a polypeptide of the invention that is expressed in a cell exhibits normal or near normal pharmacology, as defined herein. Most preferably a polypeptide of the invention that is expressed in a cell comprises the nucleotide sequence capable of encoding any one of the amino acid sequences presented in Table 1 or capable of encoding an amino acid sequence that is at least 70% identical to the amino acid sequence presented in Table 1.

According to a preferred embodiment of the present invention, a cell is selected from the group consisting of COS7-cells, a CHO cell, a LM (TK-) cell, a NIH-3T3 cell, HEK-293 cell, K-562 cell or a 1321N1 astrocytoma cell but also other transfectable cell lines.

In general, "therapeutically effective amount", "therapeutically effective dose" and "effective amount" means the amount needed to achieve the desired result or results (modulating TNF-alpha binding; treating or preventing inflammation). One of ordinary skill in the art will recognize that the potency and, therefore, an "effective amount" can vary for the various compounds that modulate TNF-alpha binding used in the invention. One skilled in the art can readily assess the potency of the compound.

As used herein, the term "compound" refers to an anti-TNF-alpha polypeptide of the present invention, a composition, or a nucleic acid capable of encoding said polypeptide or said polypeptide comprising one or more derivatised amino acids.

By "pharmaceutically acceptable" is meant a material that is not biologically or otherwise undesirable, *i.e.,* the material may be administered to an individual along with the compound without causing any undesirable biological effects or interacting in a deleterious manner with any of the other components of the pharmaceutical composition in which it is contained.

Anti-TNF-alpha polypeptides as disclosed herein is useful for treating or preventing conditions in a subject and comprises administering a pharmaceutically effective amount of a compound or composition.

Anti-TNF polypeptides of the present invention are used in a method for treating or preventing conditions relating to rheumatoid arthritis, Crohn's disease, ulcerative colitis, inflammatory bowel syndrome and multiple sclerosis in a subject and comprises administering a pharmaceutically effective amount of a compound or composition that binds TNF-alpha.

Anti-TNF-alpha polypeptides as disclosed here in are useful for treating or preventing conditions in a subject and comprises administering a pharmaceutically effective amount of a compound combination with another, such as, for example, aspirin.

The anti-TNF-alpha polypeptides as disclosed here in are useful for treating or preventing conditions relating to rheumatoid arthritis, Crohn's disease, ulcerative colitis, inflammatory bowel syndrome and multiple sclerosis in a subject and comprises administering a pharmaceutically effective amount of a compound combination with another, such as, for example, aspirin.

The present invention is not limited to the administration of formulations comprising a single compound of the invention. It is within the scope of the invention to provide combination treatments wherein a formulation is administered to a patient in need thereof that comprises more than one compound of the invention.

Conditions mediated by TNF-alpha include, but are not limited rheumatoid arthritis, Crohn's disease, ulcerative colitis, inflammatory bowel syndrome and multiple sclerosis.

A compound useful in the present invention can be formulated as pharmaceutical compositions and administered to a mammalian host, such as a human patient or a domestic animal in a variety of forms adapted to the chosen route of administration, *i.e*., orally or parenterally, by intranassally by inhalation, intravenous, intramuscular, topical or subcutaneous routes.

A compound of the present invention can also be administered using gene therapy methods of delivery. See, e.g., U.S. Patent No. 5,399,346. Using a gene therapy method of delivery, primary cells transfected with the gene for the compound of the present invention can additionally be transfected with tissue specific promoters to target specific organs, tissue, grafts, tumors, or cells.

Thus, the present compound may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitonealiy by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage.

The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compound may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, hydroxyalkyls or glycols or water-alcohol/glycol blends, in which the present compound can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compound to the skin are known to the art; for example, see Jacquet et al. (U.S. Pat. No. 4,608,392), Geria (U.S. Pat. No. 4,992,478), Smith et al. (U.S. Pat. No. 4,559,157) and Workman (U.S. Pat. No. 4,820,508).

Useful dosages of the compound can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the compound(s) in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician. Also the dosage of the compound varies depending on the target cell, tumor, tissue, graft, or organ.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

An administration regimen could include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

The invention provides for an agent that is a modulator of TNF-alpha / TNF-alpha-receptor interactions.

The candidate agent may be a synthetic agent, or a mixture of agents, or may be a natural product (*e.g.* a plant extract or culture supernatant). A candidate agent according to the disclosure includes a small molecule that can be synthesized, a natural extract, peptides, proteins, carbohydrates, lipids etc.

Candidate modulator agents from large libraries of synthetic or natural agents can be screened. Numerous means are currently used for random and directed synthesis of saccharide, peptide, and nucleic acid based agents. Synthetic agent libraries are commercially available from a number of companies including Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Combinatorial libraries are available and can be prepared. Alternatively, libraries of natural agents in the form of bacterial, fungal, plant and animal extracts are available from e.g., Pan Laboratories (Bothell, WA) or MycoSearch (NC), or are readily producible by methods well known in the art. Additionally, natural and synthetically produced libraries and agents are readily modified through conventional chemical, physical, and biochemical means.

Useful agents may be found within numerous chemical classes. Useful agents may be organic agents, or small organic agents. Small organic agents have a molecular weight of more than 50 yet less than about 2,500 daltons, preferably less than about 750, more preferably less than about 350 daltons. Exemplary classes include heterocycles, peptides, saccharides, steroids, and the like. The agents may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like.

For primary screening, a useful concentration of a candidate agent according to the invention is from about 10 mM to about 100 µM or more (*i.e*. 1 mM, 10 mM, 100 mM, 1 M etc.). The primary screening concentration will be used as an upper limit, along with nine additional concentrations, wherein the additional concentrations are determined by reducing the primary screening concentration at half-log intervals (*e.g.* for 9 more concentrations) for secondary screens or for generating concentration curves.

### High throughput screening kit

A high throughput screening kit according to the disclosure comprises all the necessary means and media for performing the detection of an agent that modulates TNF-alpha/TNF-alpha receptor interactions by interacting with TNF-alpha in the presence of a polypeptide, preferably at a concentration in the range of 1µM to 1 mM.

The kit comprises the following. Recombinant cells of the disclosure, comprising and expressing the nucleotide sequence encoding TNF-alpha, which are grown according to the kit on a solid support, such as a microtiter plate, more preferably a 96 well microtiter plate, according to methods well known to the person skilled in the art especially as described in WO 00/02045. Alternatively TNF-alpha is supplied in a purified form to be immobilized on, for example, a 96 well microtiter plate by the person skilled in the art. Alternatively TNF-alpha is supplied in the kit pre-immobilized on, for example, a 96 well microtiter plate. The TNF-alpha may be whole TNF-alpha or a fragment thereof.

Modulator agents according to the invention, at concentrations from about 1 µM to 1 mM or more, are added to defined wells in the presence of an appropriate concentration of anti-TNF-alpha polypeptide, an homologous sequence thereof, a functional portion thereof or a functional portion of an homologous sequence thereof, said concentration of said polypeptide preferably in the range of 1 µM to 1 mM. Kits may contain one or more anti-TNF-alpha polypeptide (*e.g*. one or more of a polypeptide represented by any of the SEQ ID NOs: 1 to 15 or other anti-TNF-alpha polypeptides, an homologous sequence thereof, a functional portion thereof or a functional portion of an homologous sequence thereof).

Binding assays are performed as according to the methods already disclosed herein and the results are compared to the baseline level of, for example TNF-alpha binding to an anti-TNF-alpha polypeptide, an homologous sequence thereof, a functional portion thereof or a functional portion of an homologous sequence thereof, but in the absence of added modulator agent. Wells showing at least 2 fold, preferably 5 fold, more preferably 10 fold and most preferably a 100 fold or more increase or decrease in TNF-alpha-polypeptide binding (for example) as compared to the level of activity in the absence of modulator, are selected for further analysis.

### Other Kits Useful According to the disclosure

The disclosure provides for kits useful for screening for modulators of TNF-alpha/TNF-alpha receptor binding, as well as kits useful for diagnosis of disorders characterised by dysfunction of TNF-alpha. The disclosure also provides for kits useful for screening for modulators of disorders as well as kits for their diagnosis, said disorders characterised by one or more process involving TNF-alpha. Kits useful according to the disclosure can include an isolated TNF-alpha. Alternatively, or in addition, a kit can comprise cells transformed to express TNF-alpha. In a further embodiment, a kit according to the disclosure can comprise a polynucleotide encoding TNF-alpha. In a still further embodiment, a kit according to the disclosure may comprise the specific primers useful for amplification of TNF-alpha. Kits useful according to the disclosure can comprise an isolated TNF-alpha polypeptide, a homologue thereof, or a functional portion thereof. A kit according to the disclosure can comprise cells transformed to express said polypeptide. Kits may contain more than one polypeptide, In a further embodiment, a kit according to the disclosure can comprise a polynucleotide encoding TNF-alpha. In a still further embodiment, a kit according to the disclosure may comprise the specific primers useful for amplification of a macramolecule such as, for example, TNF-alpha. All kits according to the disclosure will comprise the stated items or combinations of items and packaging materials therefore. Kits will also include instructions for use.

### EXAMPLES

The invention is illustrated by the following non-limiting example.

### Example 1: Example of Camelidae antibodies against human tumor necrosis factor alpha

### 1) Immunization and library constructions

A llama (*Llama glama*) was immunized with human TNF-alpha. For immunization, the cytokine was formulated as an emulsion with an appropriate, animal-friendly adjuvant (Specoll, CEDI Diagnostics B.V.). The antigen cocktail was administered by double-spot injections intramuscularly in the neck. The animal received 6 injections of the emulsion, containing 100 *µ*g of TNF-alpha at weekly intervals. At different time points during immunization, 10-ml blood samples were collected from the animal and sera were prepared. The induction of an antigen specific humoral immune response was verified using the serum samples in an ELISA experiment with TNF (data not shown). Five days after the last immunization, a blood sample of 150 ml was collected. From this sample, conventional and heavy-chain antibodies (HcAbs) were fractionated (Lauwereys et aL 1998) and used in an ELISA, which revealed that the HcAbs were responsible for the antigen specific humoral immune response (data not shown). Peripheral blood lymphocytes (PBLs), as the genetic source of the llama heavy chain immunoglobulins (HcAbs), were isolated from the 150-ml blood sample using a Ficoll-Paque gradient (Amersham Biosciences) yielding 5x10⁸ PBLs. The maximal diversity of antibodies is expected to be equal to the number of sampled B-lymphocytes, which is about 10 % of the number of PBLs (5x10⁷). The fraction of heavy-chain antibodies in llama is up to 20 % of the number of B-lymphocytes. Therefore, the maximal diversity of HcAbs in the 150 ml blood sample is calculated as 10⁷ different molecules. Total RNA (around 400 µg) was isolated from these cells using an acid guanidinium thiocyanate extraction (Chomczynski and Sacchi, 1987).

cDNA was prepared on 100 µg total RNA with M-MLV Reverse Transcriptase (Gibco BRL) and oligo-dT-primer or hexanucleotide random primers (Amersham Biosciences) as described before (de Haard *et al*., 1999). The cDNA was purified with a phenol/chloroform extraction combined with an ethanol precipitation and subsequently used as template to specifically amplify the VHH repertoire.

The VHH repertoire was amplified using oligo-dT primed cDNA as template with a single degenerated framework1 (FR1) primer ABL013 (5'-GAGGTBCARC**TGCAG**GASTCYGG-3'), introducing a *Pst*I restriction site (in bold), in combination with the oligo-dT primer as is described in EP01205100.9. This amplification yields two fragments of 1650 bp and 1300 bp, the latter being the product derived from the CH1-deleted HcAb genes. The smaller PCR-product was gel purified and subsequently digested with *Pst*I and *Bst*EII. The *Bst*EII-site frequently occurs within the FR4 of heavy-chain derived VHH encoding DNA-fragments.
Alternatively, the VHH-repertoire was amplified in a hinge-dependent approach using two IgG specific oligonucleotide primers. In a single PCR reaction a short (5'-AACAGTTAAGCTTCCGCTT**GCGGCCGC**GGAGCTGGGGTCTTCGCTGTGGTGCG-3') or long (5'-AACAGTTAAGCTTCCGCTT**GCGGCCGC**TGGTTGTGGTTTTGGTGTCTTGGGTT-3') hinge primer known to be specific for HcAbs was combined with the FR1-primer ABL013 3 (see above). A *Pst*I and *Not*I (bold underlined) restriction site was introduced within the FR1 and hinge primers respectively, to allow cloning. Subsequently, the DNA fragments were ligated into *Pst*I-*Bst*EII or *Pst*I*-Not*I digested phagemid vector pAX004, which is identical to pHEN1 (Hoogenboom *et al*., 1991), but encodes a carboxyterminal (His)₆- and c-myc-tag for purification and detection, respectively. The ligation mixture was desalted on a Microcon filter (YM-50, Millipore) and electroporated into *E. coli* TG1 cells to obtain a library containing 1.8×10⁷ clones. The transformed cells were grown overnight at 37°C on a single 20x20 cm plate with LB containing 100 *µ*g/ml ampicillin and 2 % glucose. The colonies were scraped from plates using 2xTY medium and stored at -80°C in 20 % glycerol.

As quality control the percentage of insert containing clones was verified on 24 clones for each library by PCR using a combination of vector based primers. This analysis revealed that 95 % of the clones contained a VHH encoding insert. The variability was examined by *Hin*fI fingerprint analysis of the amplified VHH fragment of these 24 clones, thereby showing that all clones were indeed different (data not shown).

### 2) Selection of antagonistic anti-TNF VHH's

From both libraries phage was prepared. To rescue the polyclonal phage repertoire, libraries were grown to logarithmic phase (OD600 = 0.5) at 37°C in 2xTY containing 100 *µ*g/ml ampicillin and 2 % glucose and subsequently superinfected with M13K07 helper phage for 30 minutes at 37°C. Infected cells were pelleted for 5 minutes at 4000 rpm and resuspended in 2xTY containing 100 *µ*g/ml ampicillin and 25 *µ*g/ml kanamycin. Bacteriophage was propagated by overnight growth at 37°C and 250 rpm. Overnight cultures were centrifuged for 15 minutes at 4500 rpm and phage was precipitated with one fifth volume of a [20% polyethyleneglycol 6000, 1.5 M NaCl]-solution by incubation for 30 minutes on ice. Phage was pelleted by centrifugation for 15 minutes at 4000xg and 4°C. After resuspension of the phages in PBS, cell debris was pelleted by centrifugation for 1 minute at maximal speed (15000xg) in microcentrifuge tubes. The supernatant containing the phage particles was transferred to a new tube and again phage was precipitated as described above. Phage was dissolved in PBS and separated from remaining cell debris as mentioned above. The titer of phage was determined by infection of logarithmic TG1 cells followed by plating on selective medium.

The library was selected using *in vitro* biotinylated TNF-alpha. The biotinylation was carried out as described by Magni et al (Anal Biochem 2001, 298, 181-188). The incorporation of biotin in TNF was evaluated by SDS-PAGE analysis and detection with Extravidin-alkaline phosphatase conjugate (Sigma). The functionality of the modified protein was evaluated for its ability to bind to the solid phase coated recombinant a p75 receptor.

VHH were selected by capturing biotinylated TNF-alpha (10 to 400 ng per well during 2 hours at room temperature) on streptavidin coated microtiter plates (coated with 100 µl of 10 µg/ml streptavidin during 16 hours at +4°C). Antagonistic VHH were obtained by elution with an excess of receptor, either the extracellular ligand binding domain or with cells expressing the receptor. After 2 hours incubation of phage with captured cytokine, the non-specific phage was washed away, while specific phage displaying antagonistic VHH was eluted for 30 minutes with receptor (extracellular domain of CD120b or p75; 10 µM) or with receptor displaying cells (>10⁵ KYM cells per well). High enrichments, i.e. the ratio of the number of phage eluted with receptor and those eluted by serum albumin (50 µg per well), of more than a factor of 20 suggested the successful selection of TNF-alpha specific clones. Alternatively, instead of elution with receptor a standard procedure was applied, in which a low pH causes the denaturation of VHH and / or antigen (0.1 M glycine buffer pH 2.5). Log phase growing *E. coli* cells were infected with the eluted and neutralized phage and plated on selective medium.
Individual clones were picked and grown in microtiter plate for the production of VHH in culture supernatant. ELISA screening with TNF-alpha captured on Extravidin coated plates revealed about 50% positive clones. H*in*fI-fingerprint analysis showed that 13 different clones were selected, which were grown and induced on 50 ml scale. The sequences of said clones are shown in Table 1.

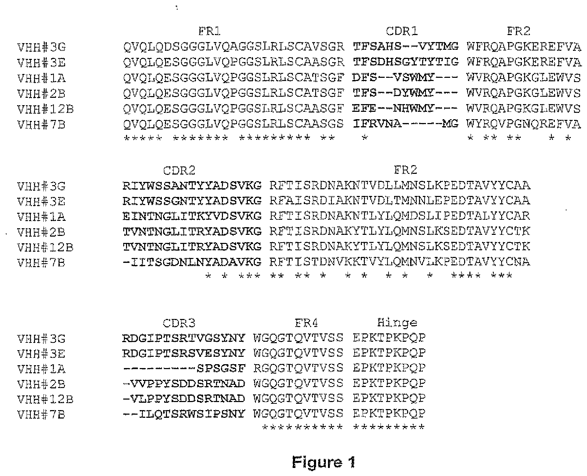

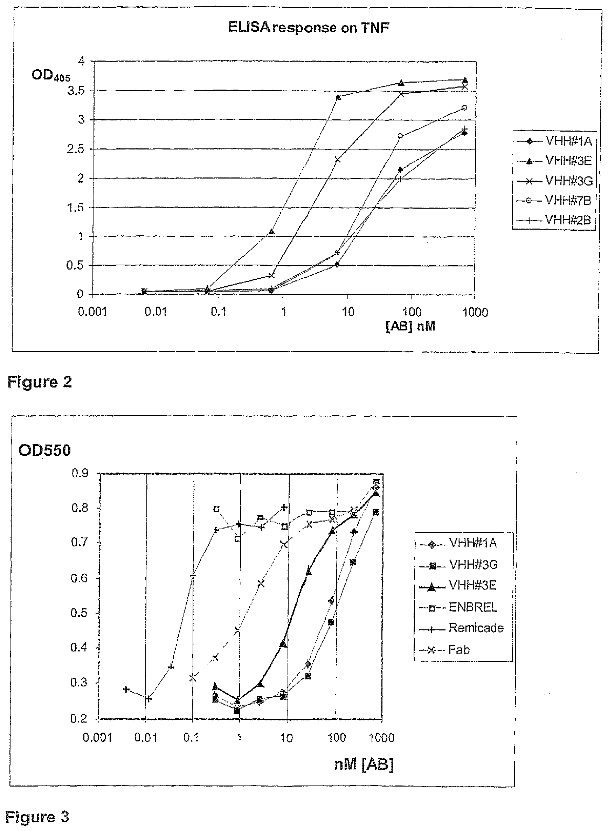

Five clones, coded VHH#1A, #2B, #3E, #3G, #7B and #12B, with different sequences (Figure 1) were characterized in more detail. VHH#3E, #3G and #7B are single-domain antibody fragments carrying the typical hydrophilic residue at position 45 (arginine) and the phenylalanine to tryptophan substitution in position 47 in FR2 thereby conferring the advantageous characteristics in terms of solubility. VHH#1A contains the hydrophobic FR2 residues typically found in double-chain antibodies of human origin or from other species, but compensating this loss in hydrophilicity by the charged arginine residue on position 103 that substitutes the conserved tryptophan residue present in VH from double-chain antibodies (WO 03/035694). A new class of humanised *Camelidae* single-domain antibodies described in this invention is represented by VHH#2B and VHH#12B, which contains the hydrophobic residues in FR2 in combination with the hydrophobic residue tryptophan at position 103. Larger amounts of antibody fragments were expressed by cultivation on 50 ml scale and purified by IMAC using TALON resin (Clontech). After dialysis against PBS to remove the eluent imidazol the amount of VHH was determined by OD280; approximately 300µg of VHH was obtained from each clone.
This material was used for determining the sensitivity of detection of (biotinylated) TNF in ELISA. For this purpose a streptavidin (10 µg/ml) coated microtiterplate was employed for capture of biotinylated TNF (1 µg/ml), VHH was diluted in 0.2 % casein / PBS and incubated for 2 hours at room temperature. Bound VHH was detected with anti-MYC mAB 9E10 (0.5 µg/ml) and anti-mouse AP conjugate (1000-fold diluted, Sigma). The results are shown in Figure 2.

### 3) Determination of Antagonistic effect in cytotoxicity assay with KYM cell line

TNF-alpha-induced cytostasis/cytotoxicity was determined by the colorimetric MTT assay as described by Vandenabeele and colleagues (Vandenabeele, P., Declercq, W., Vercammen, D., Van de Craen, M., Grooten, J., Loetscher, H., Brockhaus, M., Lesslauer, W., Fiers, W. (1992) Functional characterization of the human tumor necrosis factor receptor p75 in a transfected rat/mouse T cell hybridoma. J. Exp. Med. 176,1015-1024.). MTT (3-(4,5-cimethylthiazol-2-yl)-2,5-diphenyl tetrazolium bromide) is a pale yellow substrate that is cleaved by living cells to yield a dark blue formazan product. This process requires active mitochondria, and even freshly dead cells do not cleave significant amounts of MTT. KYM cells (Sekiguchi M, Shiroko Y, Suzuki T, Imada M, Miyahara M, Fujii G. (1985) Characterization of a human rhabdomyosarcoma cell strain in tissue culture. Boomed. Pharmacother. 39, 372-380.) were seeded in 96 well microtiterplates and cultured in the presence or absence of TNF-alpha (0.216 ng/ml or approx. 5 pM of trimer). In addition to TNF variable amounts of antibody (VHH or Remicade) were included during cultivation. For the assay MTT was added to the culture medium at a final concentration of 500 µg/ml and the plates were incubated at 37°C to achieve cleavage of MTT by mitochondrial enzymes. The formed formazon product, which appear as black, fuzzy crystals on the bottom of the well were dissolved by addition of acid isopropanol (40 nM HCl in isopropanol) or DMSO. The absorbance is measured at 570 nm.

The MTT assay (Figure 3) shows that VHH#1A, which has arginine on position 103 in combination with the human-like hydrophobic residues in FR2, has a moderate antagonistic effect (IC50 ∼100 nM). VHH#7B with the characteristic hydrophilic residues in FR2 does not prevent binding of TNF-α to its ligand in spite of its sensitive detection of the cytokine in ELISA (curve not shown). In contrast, VHH#3E and #3G with hydrophilic FR2 hallmark residues are very potent antagonistic VHH's (IC50 of 20 nM). VHH#3E and #3G have a high degree of homology and are clonally related (Harmsen et al., Mol. Immunol. 37, 579-590), but VHH#3E is more potent, probably due to the fact that it has a higher affinity than VHH#3G (Figure 2). The (chimeric) monoclonal antibody Remicade is very potent (IC50 of 80 pM), but its derived Fab fragment lost most of this efficacy (IC50 is 3 nM, 30 fold less than the intact mAB). This clearly shows the avidity effect of the interaction between the antibody and the cytokine: the mAB with two binding sites interacts more efficiently with the trimeric TNF molecule via cooperative binding. VHH fragments are strictly monovalent and therefore it was speculated that increasing the avidity by genetically fusing VHH genes might increase their antagonistic efficacy (see Example 4).
These experiments show that a new class of human-like VHH has bona fide binding and functional characteristics, thereby enabling their application for therapeutic purposes.

### Example 2: Humanization of VHH#12B and VHH#3E by site directed mutagenesis

### 1) Homology between VHH#3E / VHH#12B and human ermline heavy chain V-region DP-47

Alignment of VHH#12B and a human VH3 germline sequence (DP-47) revealed a high degree of homology:
- 4 AA changes in FR1 on position 1, 5, 28 and 30
- 5 AA changes in FR3 on position 74, 76, 83, 84 and 93
- 1 AA change in FR4 on position 108 as represented in the following sequence alignment:

DP-47 EVQLLESGGGLVQPGGSLRLSCAASGFTFS SYAMS WVRQAPGKGLEWVS AISGSGGSTYY
VHH#12B QVQLQESGGGLVQPGGSLRLSCAASGFEFE NHWMY WVRQAPGKGLEWVS TVNTNGLITRY

DP-47 ADSVKG RFTISRDNSKNTLYLQMNSLRAEDTAVYYCAK ------------- -----------
VHH#12B ADSVKG RFTISRDNAKYTLYLQMNSLKSEDTAVYYCTK VLPPYSDDSRTNAD WGQGTQVTVSS

A specific inhibitor for the TNF-alpha cytokine, with high homology to the human germline gene DP-47 was therefore an ideal candidate to further humanize and evaluate the influence of mutagenesis on inhibition capacity in ELISA.
Alignment of VHH#3E and a human VH3 germline (DP-47) revealed the presence of hydrophilic amino acid residues in FR2 of VHH#3E compared to hydrophobic residues in DP-47.

DP-47 EVQLLESGGGLVQPGGSLRLSCAASGFTFS SYAMS----- WVRQAPGKGLEWVS AISGSGGSTYY
VHH#3E QVQLQESGGGLVQPGGSLRLSCAASGRTFS DHSGYTYTIG WFRQAPGKEREFVA RIYWSSGNTYY

DP-47 ADSVKG RFTISRDNSKNTLYLQMNSLRAEDTAVYYCAK ------------- ----------

VHH#3E ADSVKG RFAISRDIAKNTVDLTMNNLEPEDTAVYYCAA RDGIPTSRSVESYNY WGQGTQVTVSS

Evaluation of the effect of substituting the hydrophilic by hydrophobic residues as present in human VH is important, since the majority of camelid VHH sequences contain hydrophilic residues.

### 2) Mutagenesis of VHH#12B

VHH#12B was mutated by using a non-PCR based site-directed mutagenesis method as described by Chen and Ruffner and commercialized by Stratagene (Quickchange site-directed mutagenesis). Plasmid DNA was used as template in combination with 2 mutagenic primers introducing the desired mutation(s). The 2 primers are each complementary to opposite strands of the template plasmid DNA. In a polymerase reaction using the *Pfu* DNA polymerase each strand is extended from the primer sequence during a cycling program using a limited number of cycles. This results in a mixture of wild type and mutated strands. Digestion with *Dpn*I results in selection of the mutated *in vitro* synthesized DNA strand, since only the template strand is sensitive for digestion. The DNA was precipitated and transformed to *E*. *coli* and analyzed for the required mutation by sequence analysis. The generated mutant VHH's and the mutagenic primers are listed in Table 2. Plasmid was prepared from mutant clones and was transformed into WK6 electrocompetent cells. A single colony was used to start an overnight culture in LB containing 2% glucose and 100 µg/ml ampicillin. This overnight culture was diluted 100-fold in 300 ml TB medium containing 100 µg/ml ampicillin, and incubated at 37°C until OD600nm= 2, when 1 mM IPTG and 5 mM MgSO₄ (final concentrations) was added and the culture was incubated for 3 more hours at 37°C.

Cultures were centrifuged for 20 minutes at 4,500 rpm at 4°C. The pellet was frozen overnight or for 1 hour at -20°C. Next, the pellet was thawed at room temperature for 40 minutes, re-suspended in 20 ml PBS/1mM EDTA/1M NaCl and shaken on ice for 1 hour. Periplasmic fraction was isolated by centrifugation for 20 minutes at 4°C at 4,500 rpm. The supernatant containing the VHH was loaded on TALON (ClonTech) and purified to homogeneity. The yield of VHH was detemined using the calculated extinction coefficient. All mutant VHH's expressed comparable to the wild type. The mutants were analyzed for their inhibition capacity in an in vitro receptor binding assay.

A microtiter plate was coated overnight at 4°C with Enbrel (Wyeth) at 2 µg/ml in PBS. The plate was washed five times with PBS-Tween and blocked for 1 hour at room temperature with PBS containing 1% casein. The plate was washed five times with PBS-Tween. Biotinylated human TNF-alpha (80 µg/ml) was pre-incubated with a dilution serie of mutant or wild type VHH#12B for 1 hr at RT and the mixture was incubated for 1 hr at room temperature in the wells of the microtiterplate. The plate was washed five times with PBS-Tween. Bound human TNF-*α* was detected using Extravidin-AP (1/1,000 dilution) and paranitrophenylphosphate (pNPP). Signals were measured after 30 minutes at 405 nm. The results are presented in Figure 4 and 5. The IC50 increased 3-fold from 66 nM (wild type) to 200 nM (mutant Q1E+Q5L+A74S+Y76N+K83R+P84A). Mutation of position T93A resulted in loss of inhibition (data not shown). The positions that still need to be humanized are: E28, E30 and Q108. However, E28 and E30 are part of the H1 canonical structure and thus part of the CDR1 according to Chothia numbering system.

The amino acid sequences of mutant VHHs are presented in Table 4 SEQ ID NOs: 17 to 19.

### 3) Mutagenesis of VHH#3E

VHH#3E was mutated by using a non-PCR based site-directed mutagenesis method as described above. The obtained mutant VHH's and the mutagenic primers are listed in Table 3.

All mutant VHH's expressed comparable to the wild type. The purified mutant VHH's were analyzed for binding in ELISA and inhibition capacity in receptor binding assay identical to the method described above.

The results of the ELISA are shown in Figure 6, those from the receptor binding assay in Figure 7.

The amino acid sequences of mutant VHHs are presented in Table 4 SEQ ID NOs 21 to 24.

### Example 3: Isolation of antagonistic VHH against mouse TNF-alpha,

### 1) Selection of anti-mouse TNF-alpha VHH

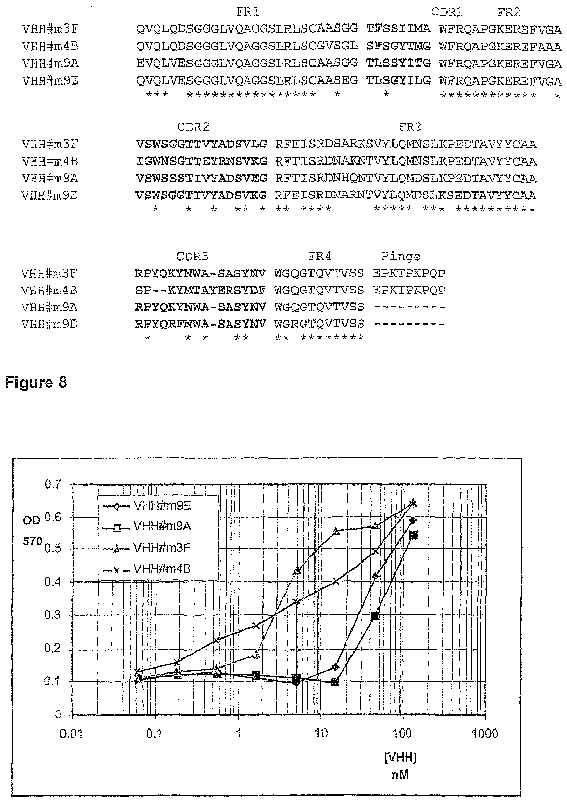

In order to perform efficacy studies in mouse models for IBD or Crohn's disease mouse TNF specific VHH were selected. Therefore a llama was immunized with mouse TNF-alpha as described in Examples 1. RNA was extracted from PBL's sampled 4 and 10 days after the last immunization, as well as from a biopsy taken from a lymph node after day 4. Total RNA was converted in either random primed or oligo-dT primed cDNA and used as template for the amplification of the VHH encoding gene segments using Ig derived primers or a combination of oligo-dT primer and a single Ig primer (see example 1). With the Ig primers a library containing 8.5 x 10⁷ clones was generated from the first PBL's, and a library with 7 x 10⁶ clones for the second PBL sample and 5.8 x 10⁸ clones for the lymph node. Using the combination of the oligo-dT primer and the Ig primer libraries from the first PBL sample were made containing 1.2 x 10⁸ clones, from the second sample of PBL's a library of 5.7 x 10⁷ clones and the lymph node derived library contained 2 x 10⁸ clones. The libraries were pooled dependent on the used combination of primers and the resulting two libraries were grown for propagation of phage as was described before. Selections were performed on biotinylated mouse TNF-alpha captured on coated streptavldin, bound phage was eluted by competition with the human receptor p75, which is known to cross-react with mouse TNF-alpha. Two distinct mouse TNF-alpha specific VHH (VHH#m3F and VHH#m9E) were selected from the library obtained by amplification with Ig derived primers, while two closely related VHH's were retrieved from the library constructed by PCR with oligo-dT primer and Ig-primer (Figure 8).

### 2) Determination antagonistic efficac in cytotoxicity assay with L929 cell line (Figure 9)

The same type of assay was applied as described in Example 1, but with the murine cell line L929. VHH#m3F and VHH#m4B (Figure 9) turned out to be 10-fold more potent then the other two VHH's.

### Examples 4: Enhancing the antagonistic efficacy by increasing the avidity using multivalent Camelidae antibodies

### 1) Antagonistic efficacy of bi-, tri- and tetravalent VHH against human and mouse TNF-alpha

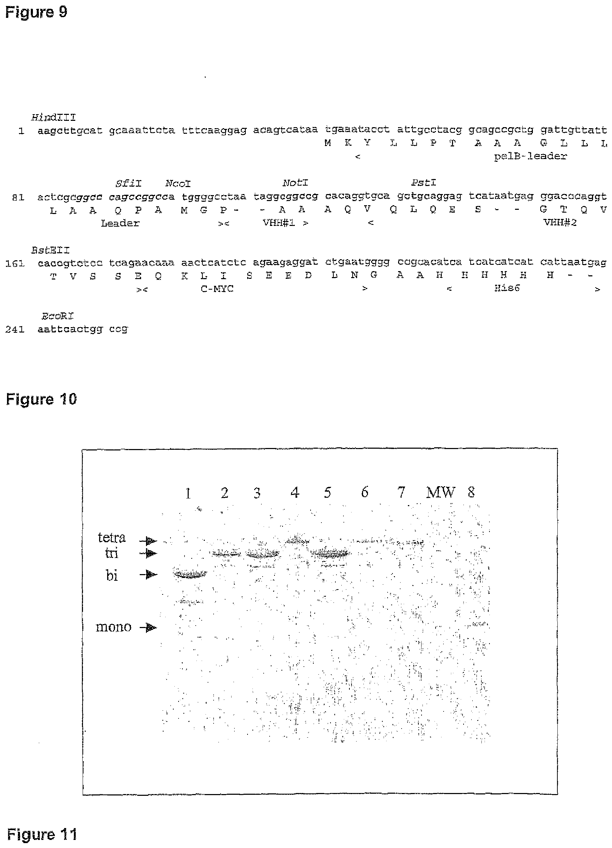

The *E. coli* production vector pAX11 (Figure 10) was designed, which allows the two-step cloning of bivalent or bispecific VHH. The carboxy terminal VHH is cloned first with *Pst*I and *Bst*EII*,* while in the second step the other VHH is inserted by *Sfi*I and *Not*I, which do not cut within the first gene fragment. The procedure avoids the enforcement of new sites by amplification and thus the risk of introducing PCR errors.
With this vector the bivalent derivative of the antagonistic anti- human TNF-alpha VHH#3E was generated. The plasmid vector encoding the bivalent VHH was used to generate a tri- and tetrameric derivative, which was accomplished by partial digestion of the plasmid with *Bst*EII, which occurs in both VHH gene segments. The linearized vector was purified from gel, subsequently de-phosphorylated and used as acceptor for cloning of the *Bst*EII fragment of approx. 350 bp that was obtained by complete digestion of the same plasmid. Ligation of the *Bst*EII fragment alone prior to addition to the vector enhances the insertion of multimeric VHH encoding gene segments. After transformation in *E. coli* TG1 the resulting clones were screened by PCR with M13Rev and M13Fwd primers; since *Bst*EII is an a-symmetric cutter (5 nt overhang) only correctly oriented inserts were obtained as was confirmed by digesting the plasmids with *Pst*I alone (350 bp) or double digesting with *Eco*RI and *Hind*III (1000 bp for bivalent (BIV 3E, SEQ ID NO: 73), 1350 bp for trivalent (TRI 3E, SEQ ID NO: 74) and 1700 bp for tetravalent (TETRA 3E, SEQ ID NO: 75), data not shown). The sequences are listed in Table 7.

The clones were grown and induced on 50 ml scale, periplasmic fractions prepared and used for IMAC purification with TALON resin. Analysis of the purified products on Coomassie stained PAGE revealed good production levels (between 2 and 10 mg per liter cell culture) of intact multivalent VHH (see Figure 11). The molecular appearance of the IMAC purified VHH was determined by gel filtration on a Superdex 75HR column and as expected the molecules with higher avidities came earlier from the column (see Figure 12).

The antagonistic efficacy was analyzed with the cell based assay using KYM cells. The cells were seeded in microtiterplates and cultured in the presence or absence of TNF-alpha (1.29 ng/ml or approx. 25 pM of trimer). The assays (Figure 13) revealed that the monovalent molecules used in this study had the poorest antagonistic characteristics, what is reflected by their IC50 values: the Fab derived from the chimeric antibody Remicade has an IC50 of 2 nM and for VHH#3E it is 12 nM (see also Figure 3). The avidity of the used molecules turned out to have a dramatic influence on the antagonistic efficacy as was observed with the bivalent IgG molecule Remicade, which is 40-fold more effective (IC50 50 pM) than the Fab. TNF-alpha is a trimeric molecule, which interacts to a dimeric receptor and therefore it can be expected that the avidity of the IgG permits the mutual binding to two epitopes on the cytokine and supports the formation of large complexes as has been described before (Santora et al, Anal.Biochem. 299, 119-129). Surprisingly, increasing the avidity of the VHH from monomer to dimer has a far more spectacular effect than observed with Remicade, since the IC50 of the dimer (30 pM) is 400 fold lower than of the monomer. Increasing the avidity even more leads to a still better antagonistic behaviour: the trimeric VHH has an IC50 of 20 pM and the tetravalent format 6 pM. All higher avidity formats of the VHH are more efficient than Remicade, while the tetravalent format is even better than Enbrel, which consists of the extracellular domain of the receptor p75 fused to the Fc of an IgG and therefore has a bivalent binding mode.

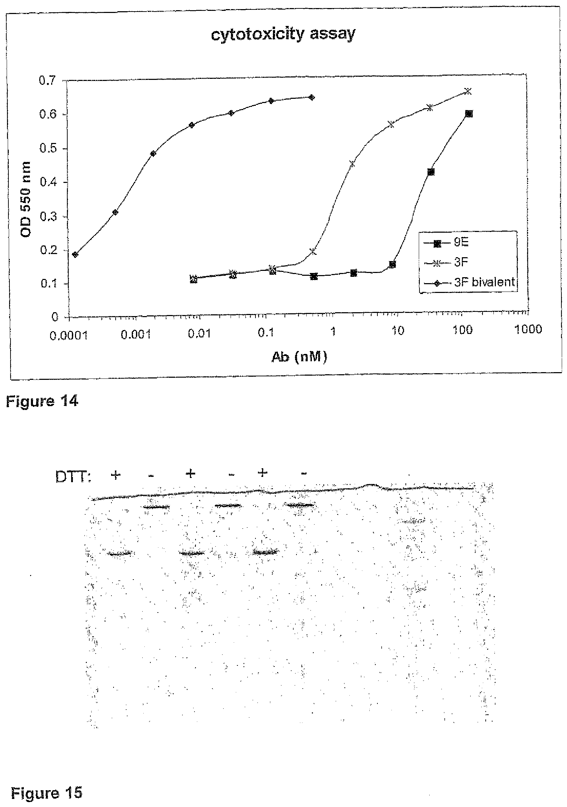

The same unexpected effect of avidity on antagonistic behaviour was observed with VHH generated against mouse TNF (Figure 14). The same type of cytotoxicity assay was performed using MTT as substrate and mouse TNF-alpha (65 pg/ml or 1.3 pM), but with the murine cell line L929, which expresses the mouse specific receptor. Three different antagonistic (monovalent) VHH were identified coded 9E and 3F, of which the first two have IC50's of 25 nM and the latter 2 nM (see also Example 3). Conversion of 3F into the bivalent format (BIV#m3F, SEQ 117 NO: 76) yielded a 1000 fold increase in IC50 (2 pM), thereby demonstrating once more that the increased avidity of the antibody leads to an unexpected improvement of the antagonistic characteristics.

### 2) Comparison with VHH-Fc fusion

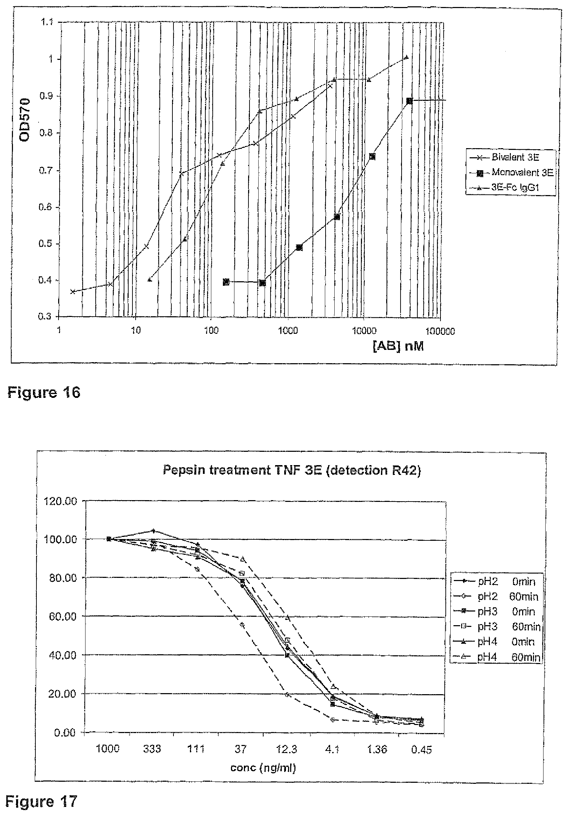

VHH#3E, directed against human TNF, was cloned via *Pst*I and *Bsf*EII in an adapted vector derived from pCDNA3, thereby generating a genetic fusion to the CH1 deleted Fc portion of human IgG1. After confirmation by sequencing, the plasmid construct was transfected to the myeloma cell line NS0. The obtained cell line was grown and the VHH-Fc fusion was secreted into the culture supernatant. The product was purified with an anti-human Fc VHH resin and analyzed on a Coomassie stained gel (Figure 15). In the presence of DTT the fusion was visible as a 45 kDa protein, in the absence of DTT the dimeric molecule with a molecular weight of 90 kDa could be observed. This dimeric product results from the linkage of two chains by two disulfide bridges, which originate from cysteine residues located in the hinge region.
The VHH-fusion was tested in the bioassay with the human cell line KYM and turned out to be 5-fold less effective than the bivalent VHH in spite of the fact that both molecules have the same avidity and that they both originate from VHH#3E (Figure 16). Probably steric hindrance by the bulky Fc tail might cause this discrepancy.

### Example 5: Calculation of homologies between anti-target-single domain antibodies of the invention

The degree of amino acid sequence homology between anti-target single domain antibodies of the invention was calculated using the Bioedit Sequence Alignment Editor. The calculations indicate the proportion of identical residues between all of the sequences as they are aligned by ClustalW. (Thompson, J.D., Higgins, D.G. and Gibson, T.J. (1994) GLUSTAL W: improving the sensitivity of progressive multiple sequence alignment through sequence weighting, position specific gap penalties and weight matrix choice. Nudeic Acids Research, submitted, June 1994). Table 8 indicates the fraction homology between anti-serum albumin VHHs of the invention. Table 9 indicates the fraction homology between anti-TNF-alpha VHHs of the invention. Table 10 indicates the percentage homology between anti-IFN-gamma VHHs of the invention.

### Example 6: Expression of a VHH-CDR3 fragment of VHH#3E

The CDR3 region of VHH#3E was amplified by using a sense primer located in the framework 4 region (Forward: CCCCTGGCCCCAGTAGTTATACG) and an anti-sense primer located in the framework 3 region (Reverse: TGTGCAGCAAGAGACGG).
In order to clone the CDR-3 fragment in pAX10, a second round PCR amplification was performed with following primers introducing the required restriction sites:
Reverse primer Sfi1: GTCCTCGCAACTGCGGCCCAGCCGGCCTGTGCAGCAAGAGACGG
Forward primer Not1: GTCCTCGCAACTGCGCGGCCGCCCCCTGGCCCCAGTAGTTATACG

The PCR reactions were performed in 50 ml reaction volume using 50pmol of each primer. The reaction conditions for the primary PCR were 11 min at 94 °C, followed by 30/60/120 sec at 94/55/72 °C for 30 cycles, and 5 min at 72°C. All reaction were performed wit 2.5 mM MgCl2, 200 mM dNTP and 1.25U AmpliTaq God DNA Polymerase (Roche Diagnostics, Brussels, Belgium).

After cleavage with Sfi1 and Not1 the PCR product was cloned in pAX10.

### Example 7: Stability testing of antibody fragments specific for human TNFα

Orally administered proteins are subject to denaturation at the acidic pH of the stomach and as well to degradation by pepsin. We have selected conditions to study the resistance of the VHH#3E to pepsin which are supposed to mimick the gastric environment. VHH#3E a VHH specific to human TNF*α* was produced as recombinant protein in E.coli and purified to homogeneity by IMAC and gelfiltration chromatography. The protein concentration after purification was determined spectrophotometrically by using the calculated molar exctinction coefficient at 280nm. Diluted solutions at 100 microgram/ml were prepared in Mcllvaine buffer (J. Biol. Chem. 49, 1921, 183) at pH 2, pH3 and 4 respectively. These solutions were subsequently incubated for 15 minutes at 37°C, prior the addition of porcine gastric mucosa pepsin at a 1/30 w/w ratio. Sixty minutes after adding the protease a sample was collected and immediately diluted 100-fold in PBS pH7,4 containing 0.1% casein to inactivate the pepsin. Seven additional 3-fold dilutions were prepared from this sample for assessing the presence of functional antibody fragment by ELISA. Identical dilutions prepared from an aliquot collected prior the addition of the protease served as a reference. In the ELISA assay biotinylated TNF*α* was captured in wells of a microtiter plate coated with neutravidin. For both the pepsin-treated and reference samples similar serial dilutions of the samples were prepared and 100 microliter of those dilutions were added to the wells. After incubation for 1 hour the plates were washed. For the detection of VHH binding to of the captured TNF*α* a polyclonal rabbit anti-VHH antiserum (R42) and an anti-rabbit IgG alkaline phosphatase conjugate was used. After washing, the plates were developed with paranitrophenyl phosphate. The data plotted in Figure 17 shows similar curves for all of the samples exposed to digestive conditions as well as for the reference samples. This indicates that the VHH#3E essentially retains its functional activity under all of the chosen conditions.

### Example 8: Oral administration of an anti-human TNFα specific VHH. in mice

An antibody solution containing the anti-human TNF*α* specific VHH#3E (100microgram per milliliter in 100-fold diluted PBS) was prepared. Three mice which were first deprived from drinking water for 12 hours and subsequently allowed to freely access the antibody solution during the next two hours. Afterwards the mice were sacrificed and their stomachs were dissected. Immediately the content of the stomachs was collected by flushing the stomach with 500microliter PBS containing 1% BSA. This flushed material was subsequently used to prepare serial three-fold dilutions, starting at a 1/5 dilution from the undiluted material. One hundred microliter of these samples was transferred to individual wells of a microtiter plater coated with human TNF*α*. After incubation for 1 hour and following extensive washing the presence of immuno-reactive material was assessed with a polyclonal rabbit anti-VHH antiserum (R42) followed by incubation with an anti-rabbit alkaline-phosphatase conjugate.The ELISA was developed with paranitrophenyl acetate. The ELISA signals obtained after 10 minutes clearly demonstrated the presence of functional VHH#3E in the gastric flushings of these mice. By comparing to the standard curve we determined the concentration of the functional antibody fragment in the gastric flushing fluid to be 1.5 , 12.6 and 8.6 microgram/ml for the three mice tested.

### Example 9: Efficacy in an animal model for IBD

### 1) Animal model of chronic collitis

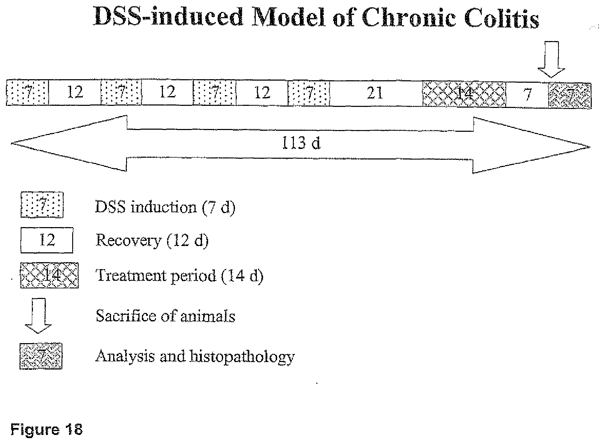

The efficacy of bivalent VHH constructs applied via various routes of administration was assessed in a DSS (dextran sodium sulfate) induced model of chronic colitis in BALB/c mice. This model was originally described by Okayasu et al. [Okayasu et al. Gastroenterology 1990; 98: 694-702] and modified by Kojouharoff et. al. [G. Kojouharoff et al. Clin. Exp. Immunol. 1997; 107: 353-8]. The animals were obtained from Charles River Laboratories, Germany, at an age of 11 weeks and kept in the animal facility until they reached a body weight between 21 and 22 g. Chronic colitis was induced in the animals by four DSS treatment cycles. Each cycle consisted of a DSS treatment interval (7 days) where DSS was provided with the drinking water at a concentration of 5 % (w/v) and a recovery interval (12 days) with no DSS present in the drinking water. The last recovery period was prolonged from 12 to 21 days to provide for an inflammation status rather representing a chronic than an acute inflammation at the time of the treatment. Subsequent to the last recovery interval the mice were randomly assigned to groups of 8 mice and treatment with the VHH-constructs was started. The treatment interval was 2 weeks. One week after the end of the treatment interval the animals were sacrificed, the intestine was dissected and histologically examined. The experimental setting is shown schematically in Figure 18.

### 2) VHH treatment schedule

During the VHH treatment period the mice (8 animals per group) were treated daily for 14 consecutive days with bivalent VHH#3F (VHH#m3F-VHH#m3F; SEQ iD No. 76) by intra-gastric or intra-venous application of 100 µg bivalent VHH 3F. An additional group of animals was treated rectally with the bivalent VHH#3F every other day for a period of 14 days. In all treatment groups a dose of 100 µg of the bivalent VHH#3F was applied at a concentration of 1 mg/ml in a buffered solution. The negative control groups received 100 µl of PBS under otherwise identical conditions. The treatment schedule is shown in Table 11.

### 3) Results

After the mice were sacrificed the body weight was determined and the colon was dissected. The length of the dissected colon was determined and the histology of the colon was assessed by Haematoxilin-Eosin (HE) stain (standard conditions). As compared to the negative controls (PBS treatment) the groups treated with bivalent nanobody 3F showed a prorogued colon length as well as an improved histological score [G. Kojouharoff et al. Clin. Exp. Immunol. 1997; 107: 353-8] thereby demonstrating efficacy of the treatment.

**Table 1: Amino acid sequence listing of the peptides directed against TNF-alpha.**

| **NAME** | **SEQ ID NO** | **SEQUENCE** |
|---|---|---|
| VHH#1A | 1 | QVQLQESGGGLVQPGGSLRLSCATSGFDFSVSWMYWVRQAPGKGLEWVS EINTNGLITKYVDSVKGRFTISRDNAKNTLYLQMDSLIPEDTALYYCAR SPSGSFRGQGTQVTVSS |
| VHH#7B | 2 | QVQLQESGGGLVQPGGSLRLSCAASGSIFRVNAMGWYRQVPGNQREFVA IITSGDNLNYADAVKGRFTISTDNVKKTVYLQMNVLKPEDTAVYYCNAI LQTSRWSIPSNYWGQGTQVTVSS |
| VHH#2B | 3 | QVQLQESGGGLVQPGGSLRLSCATSGFTFSDYWMYWVRQAPGKGLEWVS TVNTNGLITRYADSVKGRFTISRDNAKYTLYLQMNSLKSEDTAVYYCTK VVPPYSDDSRTNADWGQGTQVTVSS |
| VHH#3E | 4 | QVQLQESGGGLVQPGGSLRLSCAASGRTFSDHSGYTYTIGWFRQAPGKE REFVARIYWSSGNTYYADSVKGRFAISRDIAKNTVDLTMNNLEPEDTAV YYCAARDGIPTSRSVESYNYWGQGTQVTVSS |
| VHH#3G | 5 | QVQLQDSGGGLVQAGGSLRLSCAVSGRTFSAHSVYTMGWFRQAPGKERE FVARIYWSSANTYYADSVKGRFTISRDNAKNTVDLLMNSLKPEDTAVYY CAARDGIPTSRTVGSYNYWGQGTQVTVSS |
| VHH#10A | 6 | QVQLQESGGGLVQPGGSLRLSCAASGSIFRVNAMGWYRQVPGNQREFVA IITSSDTNDTTNYADAVKGRFTISTDNVKKTVYLQMNVLKPEDTAVYYC NAVLQTSRWSIPSNYWGQGTQVTVSS |
| VHH#2G | 7 | QVQLQDSGGGLVQAGGSLRLSCTTSGRTISVYAMGWFRQAPGKEREFVA SISGSGAITPYADSVKGRFTISRDNAKNTVYLQMNSLNPEDTAVYYCAA SRYARYRDVHAYDYWGQGTQVTVSS |
| VHH#1F | 8 | QVQLQDSGGGLVQAGGSLRLSCAASTRTFSRYVVGWFRQAPGKEREFVA TISWNGEHTYYADSVKGRYTISRDNAKNTVYLQMGSLKPEDTAVYYCAA RSFWGYNVEQRDFGSWGQGTPVTVSS |
| VHH#9C | 9 | QVQLQESGGGLVQPGGSLRLSCAASGSIFRVNAMGWYRQVPGNQREFVA IITNDTTNYADAVKGRFTISTDNVKKTVYLQMNVLKPEDTAVYYCNTVL QTSRWNIPTNYWGQGTQVTVSS |
| VHH#11E | 10 | QVQLQESGGGLVQPGGSLRLSCAASGSIFRVNAMGWYRQVPGNQREFVA IISGDTTNYADAVKGRFTISTDNVKKTVYLQMNVLESEDTAVYYCNAVL QTSRWSIPSNYWGQGTQVTVSS |
| VHH#10C | 11 | QVQLQDSGGGLVQPGGSLRLACVASGSIFSIDVMGWYRQAPGQQRELVA TITNSWTTNYADSVKGRFTISRDNAKNVVYLQMNSLKLEDTAVYYCNAR RWYQPEAWGQGTQVTVSS |
| VHH#4B | 12 | QVQLQDSGGGLVQPGGSLRLSCAASGFTFSTHWMYWVRQAPGKGLEWVS TINTNGLITDYIHSVKGRFTISRDNAKNTLYLQMNSLKSEDTAVYYCAL NQAGLSRGQGTQVTVSS |
| VHH#10D | 13 | QVQLQESGGGLVQAGGSLRLSCAASRRTFSGYAMGWFRQAPGKEREFVA VVSGTGTIAYYADSVKGRFTISRDNAENTVYLQMNSLKPEDTGLYYCAV GPSSSRWYYRGASLVDYWGKGTLVTVSS |
| VHH#12B | 14 | QVQLQESGGGLVQPGGSLRLSCAASGFEFENHWMYWVRQAPGKGLEWVS TVNTNGLITRYADSVKGRFTISRDNAKYTLYLQMNSLKSEDTAVYYCTK VLPPYSDDSRTNADWGQGTQVTVSS |
| VHH#m9A | 79 | EVQLVESGGGLVQAGGSLRLSCAASGGTLSSYITGWFRQAPGKEREFVG AVSWSSSTIVYADSVEGRFTISRDNHQNTVYLQMDSLKPEDTAVYYCAA RPYQKYNWASASASYNVWGQGTQVTVSS |
| VHH#m9E | 15 | EVQLVESGGGLVQAGGSLRLSCAASGGTLSSYITGWFRQAPGKEREFVG AVSWSSSTIVYADSVEGRFTISRDNHQNTVYLQMDSLKPEDTAVYYCAA RPYQKYNWASASASYNVWGQGTQVTVSS |
| VHH#m3F | 16 | QVQLQDSGGGLVQAGGSLRLSCAASGGTFSSIIMAWFRQAPGKEREFVG AVSWSGGTTVYADSVLGRFEISRDSARKSVYLQMNSLKPEDTAVYYCAA RPYQKYNWASASAYNVWGQGTQVTVSS |
| VHH#m4B | 80 | QVQLQDSGGGLVQAGGSLRLSCGVSGLSFSGYTMGWFRQAPGKEREFAA AIGWNSGTTEYRNSVKGRFTISRDNAKNTVYLQMNSLKPEDTAVYYCAA SPKYMTAYERSYDFWGQGTQVTVSS |
| VHH#8-29 | 81 | QVQLVESGGGLVQPGGSLRLSCAASGFAFGDSWMYWVRQAPGKGLEWVS EINTNGLITKYKDSVTGRFTISRDNAKNTLHLEMNRLKPEDTALYYCAR DPSGKLRGPGTQVTVSS |
| VHHs#8-41 | 82 | QVQLVESGGGLVQPGGPLRLSCAASGFAFGDSWMYWVRQAPGKGLEWVS EINTNGLITKYKDSVTGRFTISRDNAKNTLHLEMNRLKPEDTALYYCAR DPSGKLRGPGTQVTVSS |
| VHHs#842 | 83 | QVQLVESGGGLVQPGGSLRLSCAASGFAFGDSWMYWVRQAPGKGLEWVS EINTNGLITKYKDSVTGRFTISRDNAKNTLHLEMNRLKPEDTALYYCAR DPSGKLRGPGTQVTVSS |
| VHH#8-44 | 84 | QVQLVESGGGLVQPGGSLRLSCAASGFTFSDHWMYWVRQAPGKGLEWVS TINTNGLITNYIHSVKGRFTISRDNAKNTLYLQMNSLKSEDTAVYYCAL NQAGLSRGQGTQVTVSS |

**Table 2: List of mutagenesis reactions, mutagenic primers and templates used for mutagenesis of VHH#12B**

| **Mutation** | **Template** | **Primer sequence** |
|---|---|---|
| A74S+Y76N+ K83R+P84A | Wild type | 5'-AGA GAC AAC TCC AAG AAC ACG CTG TAT CTG CAA ATG AAC AGC CTG AGA GCT GAG GAC ACG-3' Arg Asp Asn Ser Lys Asn Thr Leu Tyr Leu Gln Met Asn Ser Leu Arg Ala Glu Asp Thr |
| Q1E+Q5L+A7 4S+Y76N+K8 3R+P84A | A74S+Y76N+ K83R+P84A | 5'-C ATG GCT GAG GTG CAG CTG CTC GAG TCT GG-3' Met Ala Glu Val Gln Leu Leu Glu Ser |
| Q1E+Q5L+A7 4S+Y76N+K8 3R+P84A+T9 3A | Q1E+Q5L+A7 4S+Y76N+K8 3R+P84A | 5'-G GAC ACG GCC GTC TAT TAC TGT GCA AAA GTA CTT C-3' Asp Thr Ala Val Tyr Tyr Cys Ala Lys Val Leu |

**Table 3: List of mutagenesis reactions, mutagenic primers and templates used for mutagenesis of VHH#3E**

| **Mutation** | **Template** | **Primer sequence** |
|---|---|---|
| F37V | Wild type | 5'-ACC TAT ACC ATT GGC TGG GTC CGC CAG GCT-3' Thr Tyr Thr Ile Gly Trp Val Arg Gln Ala |
| E44G | Wild type | 5'-CGC CAG GCT CCA GGG AAG GGG CGT GAG TTT-3' Arg Gln Ala Pro Gly Lys Gly Arg Glu Phe |
| R45L | Wild type | 5'-A GGG AAG GAG CTT GAG TTT GTA GCG CGT AT-3' Gly Lys Glu Leu Glu Phe Val Ala Arg |
| F47W | Wild type | 5'-A GGG AAG GAG CGT GAG TGG GTA GCG CGT AT-3' Gly Lys Glu Arg Glu Trp Val Ala Arg |

**Table 4: Overview of humanized and wild type anti-TNF-alpha VHH**

| **SEQ ID** | **Name** | **Sequence** |
|---|---|---|
| 17 | VHH#12B A74S+Y76N +K83R+P84 A | QVQLQESGGGLVQPGGSLRLSCAASGFEFENHWMYWVRQAPGKGLEW VSTVNTNGLITRYADSVKGRFTISRDNSKNTLYLQMNSLRAEDTAVY YCTKVLPPYSDDSRTNADWGQGTQVTVSS |
| 18 | VHH#12B Q1E+Q5L+A 74S+Y76N+ K83R+P84A | EVQLLESGGGLVQPGGSLRLSCAASGFEFENHWMYWVRQAPGKGLEW VSTVNTNGLITRYADSVKGRFTISRDNSKNTLYLQMNSLRAEDTAVY YCTKVLPPYSDDSRTNADWGQGTQVTVSS |
| 19 | VHH#12B Q1E+Q5L+A 74S+Y76N+ K83R+P84A +T93A | EVQLLESGGGLVQPGGSLRLSCAASGFEFENHWMYWVRQAPGKGLEW VSTVNTNGLITRYADSVKGRFTISRDNSKNTLYLQMNSLRAEDTAVY YCAKVLPPYSDDSRTNADWGQGTQVTVSS |
| 20 | VHH#12B Wild type | QVQLQESGGGLVQPGGSLRLSCAASGFEFENHWMYWVRQAPGKGLEW VSTVNTNGLITRYADSVKGRFTISRDNAKYTLYLQMNSLKSEDTAVY YCTKVLPPYSDDSRTNADWGQGTQVTVSS |
| 21 | VHH#3E F37V | QVQLQESGGGLVQPGGSLRLSCAASGRTFSDHSGYTYTIGWVRQAPG KEREFVARIYWSSGNTYYADSVKGRFAISRDIAKNTVDLTMNNLEPE DTAVYYCAARDGIPTSRSVESYNYWGQGTQVTVSS |
| 22 | VHH#3E E44G | QVQLQESGGGLVQPGGSLRLSCAASGRTFSDHSGYTYTIGWFRQAPG KGREFVARIYWSSGNTYYADSVKGRFAISRDIAKNTVDLTMNNLEPE DTAVYYCAARDGIPTSRSVESYNYWG |
| | | QGTQVTVSS |
| 23 | VHH#3E R45L | QVQLQESGGGLVQPGGSLRLSCAASGRTFSDHSGYTYTIGWFRQAPG KELEFVARIYWSSGNTYYADSVKGRFAISRDIAKNTVDLTMNNLEPE DTAVYYCAARDGIPTSRSVESYNYWGQGTQVTVSS |
| 24 | VHH#3E F47W | QVQLQESGGGLVQPGGSLRLSCAASGRTFSDHSGYTYTIGWFRQAPG KEREWVARIYWSSGNTYYADSVKGRFAISRDIAKNTVDLTMNNLEPE DTAVYYCAARDGIPTSRSVESYNYWGQGTQVTVSS |
| 25 | VHH#3E Wild type | QVQLQESGGGLVQPGGSLRLSCAASGRTFSDHSGYTYTIGWFRQAPG KEREFVARIYWSSGNTYYADSVKGRFAISRDIAKNTVDLTMNNLEPE DTAVYYCAARDGIPTSRSVESYNYWGQGTQVTVSS |

**Table 5: Anti-mouse serum albumin, and anti-mouse serum albumin + anti TNF-alpha VHH**

| **Name** | **SEQ ID** | **Sequence** |
|---|---|---|
| | | Anti-mouse serum albumin |
| MSA21 | 26 | QVQLQESGGGLVQPGGSLRLSCEASGFTFSRFGMTWVRQAPGKGVEWV SGISSLGDSTLYADSVKGRFTISRDNAKNTLYLQMNSLKPEDTAVYYC TIGGSLNPGGQGTQVTVSS |
| MSA24 | 27 | QVQLQESGGGLVQPGNSLRLSCAASGFTFRNFGMSWVRQAPGKEPEWV SSISGSGSNTIYADSVKDRFTISRDNAKSTLYLQMNSLKPEDTAVYYC TIGGSLSRSSQGTQVTVSS |
| MSA210 | 28 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSS |
| MSA212 | 29 | QVQLQESGGGLVQPGGSLRLTCTASGFTFRSFGMSWVRQAPGKGLEWV SAISADGSDKRYADSVKGRFTISRDNGKKMLTLDMNSLKPEDTAVYYC VIGRGSPASQGTQVTVSS |
| MSAcl6 | 85 | AVQLVESGGGLVQAGDSLRLSCVVSGTTFSSAAMGWFRQAPGKEREFV GAIKWSGTSTYYTDSVKGRFTISRDNVKNTVYLQMNNLKPEDTGVYTC AADRDRYRDRMGPMTTTDFRFWGQGTQVTVSS |
| MSAcl12 | 86 | QVKLEESGGGLVQTGGSLRLSCAASGRTFSSFAMGWFRQAPGREREFV ASIGSSGITTNYADSVKGRFTISRDNAKNTVYLQMNSLKPEDTGLCYC AVNRYGIPYRSGTQYQNWGQGTQVTVSS |
| MSAcl10 | 87 | EVQLEESGGGLVQPGGSLRLSCAASGLTFNDYAMGWYRQAPGKERDMV ATISIGGRTYYADSVKGRFTISRDNAKNTVYLQMNSLKPEDTAIYYCV AHRQTVVRGPYLLWGQGTQVTVSS |
| MSAcl14 | 88 | QVQLVESGGKLVQAGGSLRLSCAASGRTFSNYAMGWFRQAPGKEREFV AGSGRSNSYNYYSDSVKGRFTISRDNAKNTVYLQMNSLKPEDTAVYYC AASTNLWPRDRNLYAYWGQGTQVTVSS |
| MSAcl16 | 89 | EVQLVESGGGLVQAGDSLRLSCAASGRSLGIYRMGWFRQVPGKEREFV AAISWSGGTTRYLDSVKGRFTISRDSTKNAVYLQMNSLKPEDTAVYYC AVDSSGRLYWTLSTSYDYWGQGTQVTVSS |
| MSAcl19 | 90 | QVQLVEFGGGLVQAGDSLRLSCAASGRSLGIYKMAWFRQVPGKEREFV AAISWSGGTTRYIDSVKGRFTLSRDNTKNMVYLQMNSLKPDDTAVYYC AVDSSGRLYWTLSTSYDYWGQGTQVTVSS |
| MSAcl5 | 91 | EVQLVESGGGLVQAGGSLSLSCAASGRTFSPYTMGWFRQAPGKEREFL AGVTWSGSSTFYGDSVKGRFTASRDSAKNTVTLEMNSLNPEDTAVYYC AAAYGGGLYRDPRSYDYWGRGTQVTVSS |
| MScl11 | 92 | AVQLVESGGGLVQAGGSLRLSCAASGFTLDAWPIAWFRQAPGKEREGV SCIRDGTTYYADSVKGRFTISSDNANNTVYLQTNSLKPEDTAVYYCAA PSGPATGSSHTFGIYWNLRDDYDNWGQGTQVTVSS |
| MSAcl15 | 93 | EVQLVESGGGLVQAGGSLRLSCAASGFTFDHYTIGWFRQVPGKEREGV SCISSSDGSTYYADSVKGRFTISSDNAKNTVYLQMNTLEPDDTAVYYC AAGGLLLRVEELQASDYDYWGQGIQVTVSS |
| MSAcl8 | 94 | AVQLVDSGGGLVQPGGSLRLSCTASGFTLDYYAIGWFRQAPGKEREGV ACISNSDGSTYYGDSVKGRFTISRDNAKTTVYLQMNSLKPEDTAVYYC ATADRHYSASHHPFADFAFNSWGQGTQVTVSS |
| MSAcl7 | 95 | EVQLVESGGGLVQAGGSLRLSCAAYGLTFWRAAMAWFRRAPGKERELV VARNWGDGSTRYADSVKGRFTISRDNAKNTVYLQMNSLKPEDTAVYYC AAVRTYGSATYDIWGQGTQVTVSS |
| MSAcl20 | 96 | EVQLVESGGGLVQDGGSLRLSCIFSGRTFANYAMGWFRQAPGKEREFV AAINRNGGTTNYADALKGRFTISRDNTKNTAFLQMNSLKPDDTAVYYC AAREWPFSTIPSGWRYWGQGTQVTVSS |
| MSAcl4 | 97 | DVQLVESGGGWVQPGGSLRLSCAASGPTASSHAIGWFRQAPGKEREFV VGINRGGVTRDYADSVKGRFAVSRDNVKNTVYLQMNRLKPEDSAIYIC AARPEYSFTAMSKGDMDYWGKGTLVTVSS |
| | | |

| | | Anti-mouse serum albumin/anti TNF-alpha |
|---|---|---|
| MSA21/VHH#3E | 30 | QVQLQESGGGLVQPGGSLRLSCEASGFTFSRFGMTWVRQAPGKGVEWV SGISSLGDSTLYADSVKGRFTISRDNAKNTLYLQMNSLKPEDTAVYYC TIGGSLNPGGQGTQVTVSSEPKTPKPQPAAAQVQLQESGGGLVQPGGSLRL SCAASGRTFSDHSGYTYTIGWFRQAPGKEREFVARIYWSSGNTYYADSVKGRF AISRDIAKNTVDLTMNNLEPEDTAVYYCAARDGIPTSRSVESYNYWGQGTQVT VSS |
| MSA24/VHH#3E | 31 | QVQLQESGGGLVQPGNSLRLSCAASGFTFRNFGMSWVRQAPGKEPEWV SSISGSGSNTIYADSVKDRFTISRDNAKSTLYLQMNSLKPEDTAVYYC TIGGSLSRSSQGTQVTVSSEPKTPKPQPAAAQVQLQESGGGLVQPGGSLRL SCAASGRTFSDHSGYTYTIGWFRQAPGKEREFVARIYWSSGNTYYADSVKGRF AISRDIAKNTVDLTMNNLEPEDTAVYYCAARDGIPTSRSVESYNYWGQGTQVT VSS |
| MSA210/VHH#3E | 32 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQESGGGLVQPGGSLRLS CAASGRTFSDHSGYTYTIGWFRQAPGKEREFVARIYWSSGNTYYADSVKGRFA ISRDIAKNTVDLTMNNLEPEDTAVYYCAARDGIPTSRSVESYNYWGQGTQVTV SS |
| MSA212/VHH#3E | 33 | QVQLQESGGGLVQPGGSLRLTCTASGFTFRSFGMSWVRQAPGKGLEWV SAISADGSDKRYADSVKGRFTISRDNGKKMLTLDMNSLKPEDTAVYYC VIGRGSPASQGTQVTVSSEPKTPKPQPAAAQVQLQESGGGLVQPGGSLRLS CAASGRTFSDHSGYTYTIGWFRQAPGKEREFVARIYWSSGNTYYADSVKGRFA ISRDIAKNTVDLTMNNLEPEDTAVYYCAARDGIPTSRSVESYNYWGQGTQVTV SS |
| MSA21/MSA21/VHH#3E | 34 | QVQLQESGGGLVQPGGSLRLSCEASGFTFSRFGMTWVRQAPGKGVEWV SGISSLGDSTLYADSVKGRFTISRDNAKNTLYLQMNSLKPEDTAVYYC TIGGSLNPGGQGTQVTVSSEPKTPKPQPAAAQVQLQESGGGLVQPGGS LRLSCEASGFTFSRFGMTWVRQAPGKGVEWVSGISSLGDSTLYADSVK GRFTISRDNAKNTLYLQMNSLKPEDTAVYYCTIGGSLNPGGQGTQVTV SSEPKTPKPQPAAAQVQLQESGGGLVQPGGSLRLSCAASGRTFSDHSG YTYTIGWFRQAPGKEREFVARIYWSSGNTYYADSVKGRFAISRDIAKN TVDLTMNNLEPEDTAVYYCAARDGIPTSRSVESYNYWGQGTQVTVSS |
| MSA210/VHH#1A | 35 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQESGGGLVQPGGSL |
| | | RLSCATSGFDFSVSWMYWVRQAPGKGLEWVSEINTNGLITKYVDSVKG RFTISRDNAKNTLYLQMDSLIPEDTALYYCARSPSGSFRGQGTQVTVS S |
| MSA210/VHH#7B | 36 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQESGGGLVQPGGSL RLSCAASGSIFRVNAMGWYRQVPGNQREFVAIITSGDNLNYADAVKGR FTISTDNVKKTVYLQMNVLKPEDTAVYYCNAILQTSRWSIPSNYWGQG TQVTVSS |
| MSA210/VHH#2B | 37 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQESGGGLVQPGGSL RLSCATSGFTFSDYWMYWVRQAPGKGLEWVSTVNTNGLITRYADSVKG RFTISRDNAKYTLYLQMNSLKSEDTAVYYCTKVVPPYSDDSRTNADWG QGTQVTVSS |
| MSA210/VHH#3E | 38 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQESGGGLVQPGGSL RLSCAASGRTFSDHSGYTYTIGWFRQAPGKEREFVARIYWSSGNTYYA DSVKGRFAISRDIAKNTVDLTMNNLEPEDTAVYYCAARDGIPTSRSVE SYNYWGQGTQVTVSS |
| MSA210/VHH#3G | 39 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQDSGGGLVQAGGSL RLSCAVSGRTFSAHSVYTMGWFRQAPGKEREFVARIYWSSANTYYADS VKGRFTISRDNAKNTVDLLMNSLKPEDTAVYYCAARDGIPTSRTVGSY NYWGQGTQVTVSS |
| MSA21/VHH#12B | 40 | QVQLQESGGGLVQPGGSLRLSCEASGFTFSRFGMTWVRQAPGKGVEWV SGISSLGDSTLYADSVKGRFTISRDNAKNTLYLQMNSLKPEDTAVYYC TIGGSLNPGGQGTQVTVSSEPKTPKPQPAAAQVQLQESGGGLVQPGGS LRLSCAASGFEFENHWMYWVRQAPGKGLEWVSTVNTNGLITRYADSVK GRFTISRDNAKYTLYLQMNSLKSEDTAVYYCTKVLPPYSDDSRTNADW GQGTQVTVSS |
| MSA24/VHH#12B | 41 | QVQLQESGGGLVQPGNSLRLSCAASGFTFRNFGMSWVRQAPGKEPEWV SSISGSGSNTIYADSVKDRFTISRDNAKSTLYLQMNSLKPEDTAVYYC TIGGSLSRSSQGTQVTVSSEPKTPKPQPAAAQVQLQESGGGLVQPGGS LRLSCAASGFEFENHWMYWVRQAPGKGLEWVSTVNTNGLITRYADSVK GRFTISRDNAKYTLYLQMNSLKSEDTAVYYCTKVLPPYSDDSRTNADW GQGTQVTVSS |
| MSA210/VHH#12B | 42 | QVQLQESGGGLVQPGGSLRLTCTASGFTFSSFGMSWVRQAPGKGLEWV SAISSDSGTKNYADSVKGRFTISRDNAKKMLFLQMNSLRPEDTAVYYC VIGRGSPSSQGTQVTVSSEPKTPKPQPAAAQVQLQESGGGLVQPGGSL RLSCAASGFEFENHWMYWVRQAPGKGLEWVSTVNTNGLITRYADSVKG RFTISRDNAKYTLYLQMNSLKSEDTAVYYCTKVLPPYSDDSRTNADWG QGTQVTVSS |
| MSA212/VHH#12B | 43 | QVQLQESGGGLVQPGGSLRLTCTASGFTFRSFGMSWVRQAPGKGLEWV SAISADGSDKRYADSVKGRFTISRDNGKKMLTLDMNSLKPEDTAVYYC VIGRGSPASQGTQVTVSSEPKTPKPQPAAAQVQLQESGGGLVQPGGSL RLSCAASGFEFENHWMYWVRQAPGKGLEWVSTVNTNGLITRYADSVKG RFTISRDNAKYTLYLQMNSLKSEDTAVYYCTKVLPPYSDDSRTNADWG QGTQVTVSS |

**Table 6: Amino acid sequence listing of VHH's directed against human IFN-gamma.**

| **Seq. Family** | **Name** | **Seq. Id** | **Sequence** |
|---|---|---|---|
| 1 | MP3D2SRA | 44 | QVQLQDSGGGTVQAGGSLRLSCAASGRTFSDYAVGWFRQA PGKEREFVARILWTGASRSYANSVDGRFTVSTDNAKNTVY LQMNSLKPEDTAIYYCAALPSNIITTDYLRVYYWGQGTQV TVSS |
| 1 | MP3A3SR | 45 | QVQLQDSGGGTVQAGGSLRLSCAASGRTFSNYAVGWFRQA PGKEREFVARIKWSGGSRSYANSVDGRFTVSTDNAKNTVY LQMNSLKPEDTAIYYCA?LPSNIITTDYLRVYYWGQGTQV TVSS |
| 2 | MP3C5SR | 46 | QVQLQESGGGLVQAGGSLRLSCAAAGISGSVFSRTPMGWY RQAPGKQRELVAGILTSGATSYAESVKGRFTISRDNAKNT VYLQMNSLSPEDTAEYYCNTYPTWVLSWGQGTQVTVSS |
| 2 | MP3G1SR | 47 | QVQLQDSGGGLVQAGGSLRLSCAAAGISGSVFSRTPMGWY RQAPGKQRELVAGILSSGATVYAESVKGRFTISRDNAKNT VYLQMNSLSPEDTAEYYCNTYPTWVLSWGQGTQVTVSS |
| 2 | MP3G8SR | 48 | QVQLQESGGGLVQAGGSLRLSCAAAGISGSVFSRTPMGWY RQAPGKQRELVAGILSSGATAYAESVKGRFTISRDNAKNT VYLQMNSLSPEDTAEYYCNTYPTWVLSWGQGTQVTVSS |
| 3 | MP3D2BR | 49 | QVQLQESGGGLVQPGESLRLSCAASRGIFRFNAGGWYRQA PGKQRELVAFIGVDNTTRYIDSVKGRFTISRDNAKTTVYL QMNSLQPEDTAVYYCNKVPYIDWGQGTQVTVSS |
| 4 | MP3H6SRA | 50 | QVQLQESGGGLVQAGGSLRLSCAASGRTFSTYNMGWFRQA PGKEREFVAGISWNGGSIYYTSSVEGRFTISRDNAENTVY LQMNSLKPEDTGVYYCASKGRPYGVPSPRQGDYDYWGQGT QVTVSS |
| 4 | MP3B4SRA | 51 | QVQLQESGGGLVQAGGSLRLSCAASGRTFSTYNMGWFRQA PGKEREFVAGISWNGGSIYYTSSVEGRFTISRDNAENTVY LQMNSLKPEDTGVYYCASKGRPYGVPSPRQGDYDYWGQGT QVTVSS |
| 4 | MP4E4BR | 52 | QVQLQESGGGLVQAGGSLRLSCAASGRTFSIYNMGWFRQA PGKEREFVAAISWNGGSIYYTSSVEGRFTISRDNAINTVY LQMNSLKPEDTGVYYCASKGRPYGVPSPRQGEYDYWGQGT QVTVSS |
| 4 | MP4H8SR | 53 | QVQLQESGGGLVQAGGSLRLSCAASGRTFNIYNMGWFRQA PGKERDFVAAISWNGGSIYYTSSVEGRFTISRDNAENTVY LQMNSLKPEDTGVYYCASKGRPYGVPSPRQGDYDYWGQGT QVTVSS |
| 5 | MP2F6SR | 54 | QVKLEESGGGLVQAGGSLRLSCAASGRTFNNYNMGWFRQA PGKEREFVAAISWNGGSTYYDDSVKGRFTISRDNANNLVY LQMNSLNFEDTAVYYCACAANPYGIPQYRENRYDFWGQGT QVTVSS |
| 5 | MP3D1BR | 55 | QVQLQESGGGLVQAGGSLRLSCAASGRTFDNYNMGWFRQA PGKEREFVAAISWNGGSTYYDDSVKGRFTISRDNFQKLVY LQMNSLKLEDTAVYYCACAANPYGIPQYRENRYDFWGQGT QVTVSS |
| 6 | MP2B5BR | 56 | QVQLVESGGRLVQAGGSLRLSCIASGRTISDYAAGWFRQA PGKEREFLASVTWGFGSTSYADSVKGRFTISRDKAKDTVY LQMNTLEPDDTSVYYCASSPRYCAGYRCYVTASEFDSWGQ GTQVTVSS |
| 6 | MP2C1BR | 57 | QVKLEESGGRLVQAGGSLRLSCIASGRTISDYAAGWFRQA PGKEREFLASVSWGFGSTYYADSVKGRFTISRDTAKDTVY LQMNTLEPDDTSVYYCASSPRYCAGYRCYATASEFDSWGQ GTQVTVSS |
| 6 | MP4A12SR | 58 | QVQLQESGGRLVQAGGSLRLSCIASGRTISDYAAGWFRQA PGKEREFLASVTWGFGSTYYADSVKGRFTISRDKAKDTVY LQMNTLEPDDTSAYYCASSPRYCAGYRCYVTASEFDSWGP GTQVTVSS |
| 7 | MP3F4SRA | 59 | QVQLQDSGGGLVQAGDSLRLSCAASGRSFSSYGMGWFRQA PGKEHEFVAGIWRSGVSLYYTDSVKGRFTISRDDAKMTVS LQMNSLKPEDTAVYYCAAEATFPTWSRGRFADYDYRGQGT QVTVSS |
| 7 | MP3D3BR | 60 | QVQLQESGGGLVQAGDSLRLSCTASGRSFSSYGMGWFRQA PGKDHEFVAGIWRSGVSLYYADSVKGRFTISRDDAKMTVS LQMNGLKPEDTAVYYCAAEATFPTWNRGTFADYDYRGQGT QVTVSS |
| 7 | MP3E5BR | 61 | QVQLQESGGGLVQAGDSLRLSCAASGRSFSSYGMGWFRQA PGKEHEFVAGIWRSGVSLYYADSVKGRFTISRDDAKMTVS LQMNGLKPEDTAVYYCAAEATFPTWNRGSFADYDYRGQGT QVTVSS |
| 7 | MP3C7SRA | 62 | QVQLQESGGGLVQAGDSLRLSCAASGRSFSSYGMGWFRQA PGKEHEFVAGIWRSGVSLYYADSVKGRFTISRDDAKMTVS LQMNSLKPEDTAVYYCAAEATFPTWNRGRFADYDYSGQGT QVTVSS |
| 8 | MP2F1BR | 63 | AVQLVESGGGLVQTGDSLRLSCVASGGTFSRYAMGWFRQA PGKEREFVARIGYSGRSISYATSVEGRFAISRDNAKNTVY LQMNSLKPEDTAVYYCASLVSGTLYQADYWGQGTQVTVSS |
| 8 | MP2C5BR | 64 | QVQLVESGGGLVQTGDSLRLSCVASGGTFSRYAMGWFRQP PGKERDFVARIGYSGQSISYATSVEGRFAISRDNAKNTVY LQMNSLKPEDTAVYYCASLVSGTLYKPNYWGQGTQVTVSS |
| 9 | MP2C10BR | 65 | QVKLEESGGGLVQAGGSLRLSCAASGLTYTVGWFRQAPGK EREFVAAISWSGGSALYADSVKGRFTISRDNAKNTVYLQM GSLEPEDTAYYSCAAPGTRYYGSNQVNYNYWGQGTQVTVS S |
| 9 | MP2G5SR | 66 | QVKLEESGGGLVQAGDSLRLSCAASGLTYTVGWFRQAPGK EREFVAAIDWSGGSALYADSVKGRFTISRDNTKNTVYLQM GSLEPEDTAVYWCAAPGTRYHGRNQVNYNYWGQGTQVTVS S |
| 10 | MP3B1SRA | 67 | QVQLQESGGGLVQPGGSLRLSCAASGFTSSNYAMSWVRQA PGKGLEWVSSINSRTGSITYADSVKGRFTITLDNAKNTLY LQMNSLKPEDTAVYYCASRVDDRVSRGQGTQVTVSS |
| 11 | MP2F10SR | 68 | QVQLVESGGGLVQAGGSLRLSCAASGRTISSFRMGWFRRA PGEEREFVAFVRSNGTSTYYADSVEGRFTITRDNAKNTVY LRMDSLKPEDTAVYYCAAATRDYGGSFDYWGQGTQVTVSS |
| 11 | MP3A7SRA | 69 | QVQLQDSGGGLVQAGGSLRLSCAASGRTFSSFRMGWFRRA PGEEREFVAFVRSNGTSTYYADSVEGRFTITRDNAKNTVY LRMDSLKPEDTAVYYCAAATRDYGGSFDYWGQGTQVIVSS |
| 12 | MP4C10SR | 70 | QVQLQESGGGLVQPGGSLRLSCAASGFTVSNYAMSWVRQP PGKGIEWVSSINNRNDHITYADSVKGRFTIARDNANNILY LQMNSLKPEDTAVYYCASRVDDRVSRGQGTQVTVSS |
| 13 | MP4D5BR | 71 | QVQLQDSGGGLVQPGGSLRLSCAASGRTFSSYGMAWFRQA PGKERELVVAINRSGGATSYATSVRGRFTISRDNAKNTMY LQMNSLNPEDTAVYYCAARDPTRTYSSYFEYTYWGQGTQV TVSS |
| 14 | MP3F1SRA | 72 | QVQLQESGGGLVQAGGSLTLSCVASGRTISDYAVGWFRQA PGKEREFVASISWGGGFTAFADSMKGRFTISRDNAKNTVY LQTHTLEPDDTSVYYCASSRRYCTGYRCYATASEFDSWGQ GTQVTVSS |

**Table 7: Sequences of bivalent (BIV 3E, BIV#m3F), trivalent (TRI3E) or tetravalent (TETRA 3E) VHH directed against TNF-alpha.**

| **Name** | **SEQ ID** | **Sequence** |
|---|---|---|
| | | **With linker sequence (underlined)** |
| BIV 3E | 73 | QVQLQDSGGGLVQAGGSLRLSCAASGGTFSSIIMAWFRQAPGKEREFVGAVSWSG GTTVYADSVLGRFEISRDSARKSVYLQMNSLKPEDTAVYYCAARPYQKYNWASAS YNVWGQGTQVTVSSEPKTPKPQPAAAQVQLQDSGGGLVQAGGSLRLSCAASGGTF SSIIMAWFRQAPGKEREFVGAVSWSGGTTVYADSVLGRFEISRDSARKSVYLQMN SLKPEDTAVYYCAARPYQKYNWASASYNVWGQGTQVTVSS |
| TRY 3E | 74 | QVQLQESGGGLVQPGGSLRLSCAASGRTFSDHSGYTYTIGWFRQAPGKEREFVAR IYWSSGNTYYADSVKGRFAISRDIAKNTVDLTMNNLEPEDTAVYYCAARDGIPTS RSVESYNYWGQGTQVTVSSEPKTPKPQPAAAQVQLQESGGGLVQPGGSLRLSCAA SGRTFSDHSGYTYTIGWFRQAPGKEREFVARIYWSSGNTYYADSVKGRFAISRDI AKNTVDLTMNNLEPEDTAVYYCAARDGIPTSRSVESYNYWGQGTQVTVSSEPKTP KPQPAAAQVQLQESGGGLVQPGGSLRLSCAASGRTFSDHSGYTYTIGWFRQAPGK EREFVARIYWSSGNTYYADSVKGRFAISRDIAKNTVDLTMNNLEPEDTAVYYCAA RDGIPTSRSVESYNYWGQGTQVTVSS |
| TETRA 3E | 75 | QVQLQESGGGLVQPGGSLRLSCAASGRTFSDHSGYTYTIGWFRQAPGKEREFVAR IYWSSGNTYYADSVKGRFAISRDIAKNTVDLTMNNLEPEDTAVYYCAARDGIPTS RSVESYNYWGQGTQVTVSSEPKTPKPQPAAAQVQLQESGGGLVQPGGSLRLSCAA SGRTFSDHSGYTYTIGWFRQAPGKEREFVARIYWSSGNTYYADSVKGRFAISRDI AKNTVDLTMNNLEPEDTAVYYCAARDGIPTSRSVESYNYWGQGTQVTVSSEPKTP KPQPAAAQVQLQESGGGLVQPGGSLRLSCAASGRTFSDHSGYTYTIGWFRQAPGK EREFVARIYWSSGNTYYADSVKGRFAISRDIAKNTVDLTMNNLEPEDTAVYYCAA RDGIPTSRSVESYNYWGQGTQVTVSSEPKTPKPQPAAAQVQLQESGGGLVQPGGS LRLSCAASGRTFSDHSGYTYTIGWFRQAPGKEREFVARIYWSSGNTYYADSVKGR FAISRDIAKNTVDLTMNNLEPEDTAVYYCAARDGIPTSRSVESYNYWGQGTQVTV SS |
| BIV#m 3F | 76 | QVQLQDSGGGLVQAGGSLRLSCAASGGTFSSIIMAWFRQAPGKEREFVGAVSWSG GTTVYADSVLGRFEISRDSARKSVYLQMNSLKPEDTAVYYCAARPYQKYNWASAS YNVWGQGTQVTVSSEPKTPKPQPAAAQVQLQDSGGGLVQAGGSLRLSCAASGGTF SSIIMAWFRQAPGKEREFVGAVSWSGGTTVYADSVLGRFEISRDSARKSVYLQMN SLKPEDTAVYYCAARPYQKYNWASASYNVWGQGTQVTVSS |
| | | **Without linker sequence** |
| BIV#3 Edir | 77 | QVQLQDSGGGLVQAGGSLRLSCAASGGTFSSIIMAWFRQAPGKEREFVGAVSWSG GTTVYADSVLGRFEISRDSARKSVYLQMNSLKPEDTAVYYCAARPYQKYNWASAS YNVWGQGTQVTVSSQVQLQDSGGGLVQAGGSLRLSCAASGGTFSSIIMAWFRQAP GKEREFVGAVSWSGGTTVYADSVLGRFEISRDSARKSVYLQMNSLKPEDTAVYYC AARPYQKYNWASASYNVWGQGTQVTVSS |
| BIV#12 Bdlr | 78 | QVQLQESGGGLVQPGGSLRLSCAASGFEFENHWMYWVRQAPGKGLEWVSTVNTNG LITRYADSVKGRFTISRDNAKYTLYLQMNSLKSEDTAVYYCTKVLPPYSDDSRTN ADWGQGTQVTVSSQVQLQESGGGLVQPGGSLRLSCAASGFEFENHWMYWVRQAPG KGLEWVSTVNTNGLITRYADSVKGRFTISRDNAKYTLYLQMNSLKSEDTAVYYCT KVLPPYSDDSRTNADWGQGTQVTVSS |

**Table 8: Fractional homologies between the amino acid sequences of anti-mouse serum albumin VHHs.**

| **SEQ** | **MESA21** | **MSA24** | **MSA210** | **MSA212** |
|---|---|---|---|---|
| **MSA21** | 1.000 | 0.834 | 0.800 | 0.782 |
| **MSA24** | --- | 1.000 | 0.782 | 0.791 |
| **MSA210** | --- | --- | 1.000 | 0.903 |
| **MSA212** | --- | --- | --- | 1.000 |

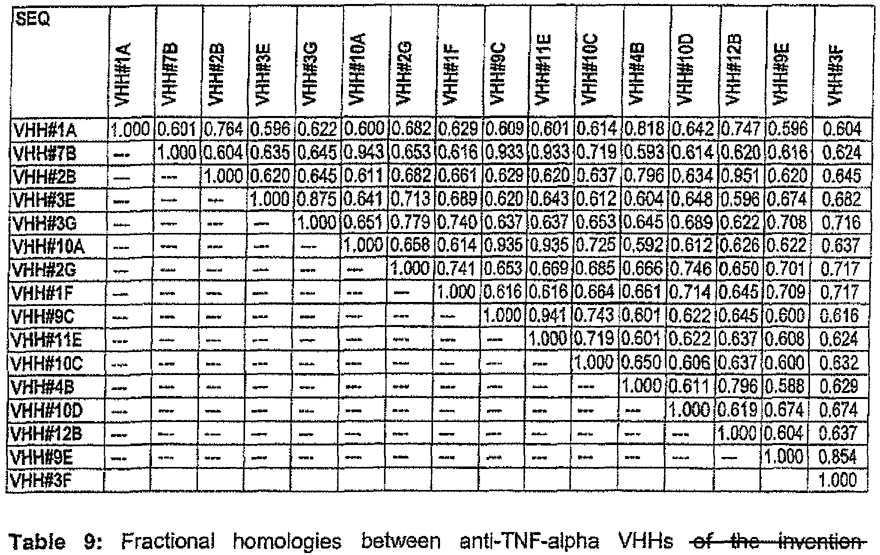

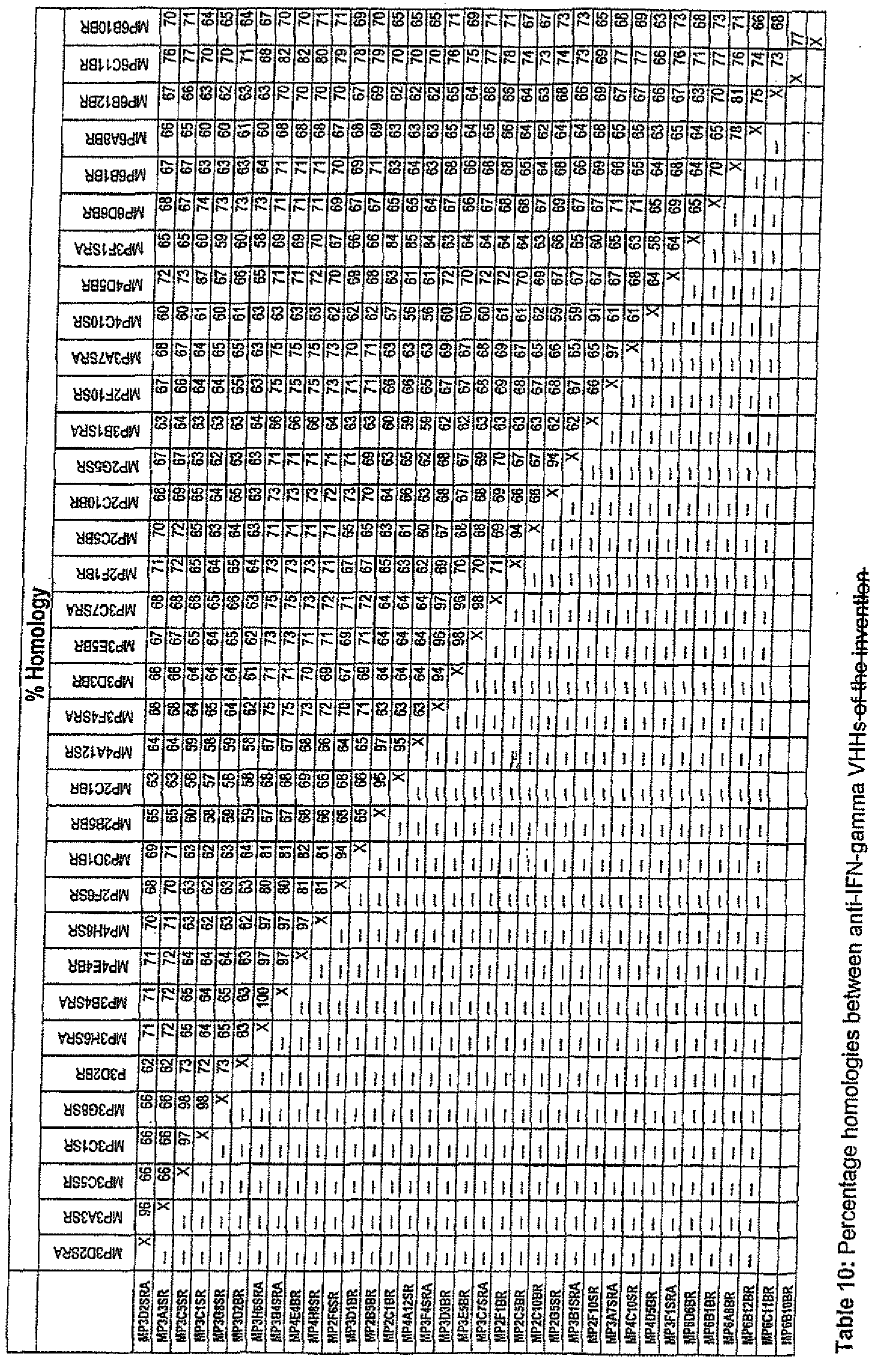

**Table 11: Treatment schedule**

| **Group** | **Animals** | **Description** | **Schedule** |
|---|---|---|---|
| 1 | 8 | negative control 1 ip | daily 100 µl PBS i.p. + |
| 2 | 8 | negative control 2 rectal | every other day 100 µl PBS rectal for 2 weeks |
| 3 | 8 | negative control 3 intragastric | daily 100 µl PBS intragastric for 14 consecutive days |
| 4 | 8 | positive control 1 dexamethasone | 5 µg i.p. for 7 consecutive days |
| 5 | 8 | positive control 2 IL 10 expressing l.lactis | applied orally once per day for 14 consecutive days |
| 6 | 8 | bivalent VHH 3F intra-gastric | daily 100 µg bivalent VHH 3F₂ intragastric on 14 consecutive days |
| 7 | 8 | bivalent VHH 3F i.p. | daily 100 µg bivalent VHH 3F i.p. for 14 consecutive days |
| 8 | 8 | bivalent VHH 3F rectally | 100 µg bivalent VHH 3F rectally in 100 µl PBS every other day for two weeks |

## Claims

1. An anti-TNF-alpha polypeptide comprising:
- at least two anti-TNF-alpha single domain antibodies; and
- at least one single domain antibody directed against a serum protein, wherein said serum protein is any of serum albumin, serum immunoglobulins, thyroxine-binding protein, transferrin, or fibrinogen.

2. The anti-TNF-alpha polypeptide according to claim 1 comprising:
- two anti-TNF-alpha single domain antibodies; and
- one single domain antibody directed against a serum protein, wherein said serum protein is any of serum albumin, serum immunoglobulins, thyroxine-binding protein, transferrin, or fibrinogen.

3. The anti-TNF-alpha polypeptide according to claim 1 or 2, comprising at least one anti-TNF-alpha single domain antibody comprising
(a) a sequence represented by any of SEQ ID NOs: 1 to 16 and 79 to 84,
(b) a homologous sequence which presents a sequence identity of more than 70% with the parent sequence represented by any of SEQ ID NOs: 1 to 16 and 79 to 84.

4. The anti-TNF-alpha polypeptide according to any one of claims 1 to 3 comprising at least one anti-TNF-alpha single domain antibody comprising
(a) a sequence represented by SEQ ID NO: 1,
(b) a homologous sequence which presents a sequence identity of more than 70% with the parent sequence represented by SEQ ID NO: 1.

5. The anti-TNF-alpha polypeptide according to any one of claims 1 to 4 comprising at least one anti-TNF-alpha single domain antibody comprising
(a) a sequence represented by SEQ ID NO: 1,
(b) a homologous sequence which presents a sequence identity of 80% with the parent sequence represented by SEQ ID NO: 1.

6. The anti-TNF-alpha polypeptide according to any one of claims 1 to 5 in which the at least one single domain antibody directed against a serum protein is directed against serum albumin.

7. The anti-TNF-alpha polypeptide according to any of claims 1 to 3 and 6, in which the single domain antibodies of the anti-TNF-alpha polypeptide which are directed against TNF-alpha are of the same sequence.

8. The anti-TNF-alpha polypeptide according to any of claims 1 to 7, wherein at least one single domain antibody is a humanized *Camelidae* VHH.

9. A nucleic acid encoding an anti-TNF-alpha polypeptide according to any of claims 1 to 8.

10. An anti-TNF-alpha polypeptide of any of claims 1 to 8, or a nucleic acid according to claim 9 for use in a method for treating and/or preventing and/or alleviating disorders relating to inflammatory processes.

11. An anti-TNF-alpha polypeptide or nucleic acid for use according to claim 10, wherein said disorders are any of inflammation, rheumatoid arthritis, Crohn's disease, ulcerative colitis, inflammatory bowel syndrome, multiple sclerosis, Addison's disease, Autoimmune hepatitis, Autoimmune parotitis, Diabetes Type 1, Epididymitis, Glomerulonephritis, Graves' disease, Guillain-Barre syndrome, Hashimoto's disease, Hemolytic anemia, Systemic lupus erythematosus, Male infertility, Myasthenia Gravis, Pemphigus, Psoriasis, Rheumatic fever, Sarcoidosis, Scleroderrna, Sjogren's syndrome, Spondyloarthropathies, Thyroiditis, and Vasculitis.

12. A composition comprising a nucleic acid according to claim 9 or an anti-TNF-alpha polypeptide of any of claims 1 to 8, and a suitable pharmaceutical vehicle.

13. Use of an anti-TNF-alpha polypeptide of any of claims 1 to 8 or a nucleic acid according to claim 9, or a composition according to claim 12 for the preparation of a medicament for treating and/or preventing and/or alleviating disorders relating to inflammatory reactions.

## Patentansprüche

1. Anti-TNF-alpha-Polypeptid, umfassend:
- wenigstens zwei Anti-TNF-alpha-Einzeldomänen-Antikörper; und
- wenigstens einen Einzeldomänen-Antikörper, der gegen ein Serumprotein gerichtet ist, wobei das Serumprotein irgendeines aus Serumalbumin, Serumimmunglobulinen, Thyroxin-bindendem Protein, Transferrin oder Fibrinogen ist.

2. Anti-TNF-alpha-Polypeptid gemäß Anspruch 1, umfassend:
- zwei Anti-TNF-alpha-Einzeldomänen-Antikörper; und
- einen Einzeldomänen-Antikörper, der gegen ein Serumprotein gerichtet ist, wobei das Serumprotein irgendeines aus Serumalbumin, Serumimmunglobulinen, Thyroxin-bindendem Protein, Transferrin oder Fibrinogen ist.

3. Anti-TNF-alpha-Polypeptid gemäß Anspruch 1 oder 2, welches wenigstens einen Anti-TNF-alpha-Einzeldomänen-Antikörper umfasst, umfassend
(a) eine Sequenz, repräsentiert durch irgendeine der SEQ ID NOs: 1 bis 16 und 79 bis 84,
(b) eine homologe Sequenz, die eine Sequenzidentität von mehr als 70% zu der Elternsequenz, repräsentiert durch irgendeine der SEQ ID NOs: 1 bis 16 und 79 bis 84, aufweist.

4. Anti-TNF-alpha-Polypeptid gemäß irgendeinem der Ansprüche 1 bis 3, welches wenigstens einen Anti-TNF-alpha-Einzeldomänen-Antikörper umfasst, umfassend
(a) eine Sequenz, repräsentiert durch SEQ ID NO: 1,
(b) eine homologe Sequenz, die eine Sequenzidentität von mehr als 70% zu der Elternsequenz, repräsentiert durch SEQ ID NO: 1, aufweist.

5. Anti-TNF-alpha-Polypeptid gemäß irgendeinem der Ansprüche 1 bis 4, welches wenigstens einen Anti-TNF-alpha-Einzeldomänen-Antikörper umfasst, umfassend
(a) eine Sequenz, repräsentiert durch SEQ ID NO: 1,
(b) eine homologe Sequenz, die eine Sequenzidentität von 80% zu der Elternsequenz, repräsentiert durch SEQ ID NO: 1, aufweist.

6. Anti-TNF-alpha-Polypeptid gemäß irgendeinem der Ansprüche 1 bis 5, wobei der wenigstens eine gegen ein Serumprotein gerichtete Einzeldomänen-Antikörper gegen Serumalbumin gerichtet ist.

7. Anti-TNF-alpha-Polypeptid gemäß irgendeinem der Ansprüche 1 bis 3 und 6, wobei die Einzeldomänen-Antikörper des Anti-TNF-alpha-Polypeptids, die gegen TNF-alpha gerichtet sind, die gleiche Sequenz haben.

8. Anti-TNF-alpha-Polypeptid gemäß irgendeinem der Ansprüche 1 bis 7, wobei wenigstens ein Einzeldomänen-Antikörper ein humanisierter *Camelidae*-VHH ist.

9. Nukleinsäure, die ein Anti-TNF-alpha-Polypeptid gemäß irgendeinem der Ansprüche 1 bis 8 codiert.

10. Anti-TNF-alpha-Polypeptid gemäß irgendeinem der Ansprüche 1 bis 8 oder Nukleinsäure gemäß Anspruch 9 zur Verwendung in einem Verfahren zur Behandlung und/oder Prävention und/oder Linderung von Störungen, die entzündliche Prozesse betreffen.

11. Anti-TNF-alpha-Polypeptid oder Nukleinsäure zur Verwendung gemäß Anspruch 10, wobei die Störungen irgendwelche unter Entzündung, rheumatoider Arthritis, Crohn-Krankheit, ulcerativer Kolitis, entzündlichem Darmsyndrom, Multipler Sklerose, Addison-Krankheit, Autoimmun-Hepatitis, Autoimmun-Parotitis, Diabetes Typ 1, Epididymitis, Glomerulonephritis, Graves-Erkrankung, Guillain-Barre-Syndrom, Hashimoto-Erkrankung, hämolytischer Anämie, systemischem Lupus erythematodes, männlicher Infertilität, Myasthenia gravis, Pemphigus, Psoriasis, rheumatischem Fieber, Sarkoidose, Sklerodermie, Sjögren-Syndrom, Spondyloarthropathien, Thyreoiditis und Vaskulitis sind.

12. Zusammensetzung, welche eine Nukleinsäure gemäß Anspruch 9 oder ein Anti-TNF-alpha-Polypeptid gemäß irgendeinem der Ansprüche 1 bis 8 und ein geeignetes pharmazeutisches Vehikel umfasst.

13. Verwendung eines Anti-TNF-alpha-Polypeptids gemäß irgendeinem der Ansprüche 1 bis 8 oder einer Nukleinsäure gemäß Anspruch 9 oder einer Zusammensetzung gemäß Anspruch 12 für die Herstellung eines Medikaments zur Behandlung und/oder Prävention und/oder Linderung von Störungen, die entzündliche Reaktionen betreffen.

## Revendications

1. Polypeptide anti-TNF-alpha comprenant :
- au moins deux anticorps à domaine unique anti-TNF-alpha ; et
- au moins un anticorps à domaine unique dirigé contre une protéine sérique, dans lequel ladite protéine sérique est l'un quelconque de l'albumine sérique, des immunoglobulines sériques, de la protéine liant la thyroxine, de la transferrine ou du fibrinogène.

2. Polypeptide anti-TNF-alpha selon la revendication 1 comprenant :
- deux anticorps à domaine unique anti-TNF-alpha ; et
- un seul anticorps à domaine unique dirigé contre une protéine sérique, où ladite protéine sérique est l'un quelconque de l'albumine sérique, des immunoglobulines sériques, de la protéine liant la thyroxine, de la transferrine ou du fibrinogène.

3. Polypeptide anti-TNF-alpha selon la revendication 1 ou 2, comprenant au moins un anticorps à domaine unique anti-TNF-alpha comprenant
(a) une séquence représentée par l'une quelconque des SEQ ID NOs: 1 à 16 et 79 à 84,
(b) une séquence homologue qui présente une identité de séquence de plus de 70 % avec la séquence parente représentée par l'une quelconque des SEQ ID NOs: 1 à 16 et 79 à 84.

4. Polypeptide anti-TNF-alpha selon l'une quelconque des revendications 1 à 3 comprenant au moins un anticorps à domaine unique anti-TNF-alpha comprenant
(a) une séquence représentée par SEQ ID NO: 1,
(b) une séquence homologue qui présente une identité de séquence de plus de 70 % avec la séquence parente représentée par SEQ ID NO: 1.

5. Polypeptide anti-TNF-alpha selon l'une quelconque des revendications 1 à 4 comprenant au moins un anticorps à domaine unique anti-TNF-alpha comprenant
(a) une séquence représentée par SEQ ID NO: 1,
(b) une séquence homologue qui présente une identité de séquence de 80 % avec la séquence parente représentée par SEQ ID NO: 1.

6. Polypeptide anti-TNF-alpha selon l'une quelconque des revendications 1 à 5 dans lequel le au moins un anticorps à domaine unique dirigé contre une protéine sérique est dirigé contre l'albumine sérique.

7. Polypeptide anti-TNF-alpha selon l'une quelconque des revendications 1 à 3 et 6, dans lequel les anticorps à domaine unique du polypeptide anti-TNF-alpha qui sont dirigés contre le TNF-alpha sont de la même séquence.

8. Polypeptide anti-TNF-alpha selon l'une quelconque des revendications 1 à 7, dans lequel au moins un anticorps à domaine unique est un VHH de *Camelidae* humanisé.

9. Acide nucléique codant pour un polypeptide anti-TNF-alpha selon l'une quelconque des revendications 1 à 8.

10. Polypeptide anti-TNF-alpha selon l'une quelconque des revendications 1 à 8, ou acide nucléique selon la revendication 9 pour une utilisation dans un procédé pour le traitement et/ou la prévention et/ou le soulagement de troubles associés à des processus inflammatoires.

11. Polypeptide anti-TNF-alpha ou acide nucléique pour une utilisation selon la revendication 10, dans lequel lesdits troubles sont l'un quelconque parmi une inflammation, une polyarthrite rhumatoïde, la maladie de Crohn, une colite ulcéreuse, un syndrome de l'intestin inflammatoire, une sclérose en plaques, la maladie d'Addison, une hépatite auto-immune, une parotidite auto-immune, un diabète de type 1, une épididymite, une glomérulonéphrite, la maladie de Graves, le syndrome de Guillain-Barré, la maladie de Hashimoto, une anémie hémolytique, un lupus érythémateux systémique, une infertilité masculine, une myasthénie gravis, un pemphigus, un psoriasis, un rhumatisme articulaire aigu, une sarcoïdose, une sclérodermie, le syndrome de Sjögren, des spondyloarthropathies, une thyroïdite, et une vascularite.

12. Composition comprenant un acide nucléique selon la revendication 9 ou un polypeptide anti-TNF-alpha selon l'une quelconque des revendications 1 à 8, et un véhicule pharmaceutique approprié.

13. Utilisation d'un polypeptide anti-TNF-alpha selon l'une quelconque des revendications 1 à 8 ou d'un acide nucléique selon la revendication 9, ou d'une composition selon la revendication 12 pour la préparation d'un médicament destiné au traitement et/ou à la prévention et/ou au soulagement de troubles associés à des réactions inflammatoires.
